# EUROPEAN PATENT APPLICATION

(11) **EP 4 455 148 A1**
(43) Date of publication of application: **30.10.2024**
(21) Application number: 22910019.3
(22) Date of filing: 20.12.2022
(51) Int. Cl.: C07D 495/04, A61K 31/407, A61P 29/00, A61P 35/00

(54) **PREPARATION FOR THIOPHENE DERIVATIVE**

(30) Priority: 21.12.2021 CN 202111575602
(71) Applicant: Ganzhou Hemay Pharmaceutical, Co., Ltd, Jiangxi Province (CN)
(72) Inventor: ZENG, Guanghuai, Ganzhou, Jiangxi 341600 (CN); CHANG, Yukun, Ganzhou, Jiangxi 341600 (CN)
(74) Representative: Cabinet Netter
(86) International application number: PCT/CN2022/140411
(87) International publication number: WO 2023/116707

(57) **Abstract**

Disclosed is a process for preparing a compound of formula (VII), 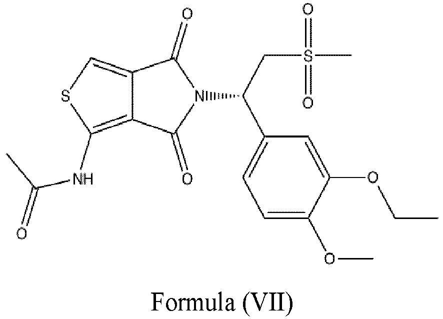 comprises subjecting a compound of formula (VI) to a condensation reaction

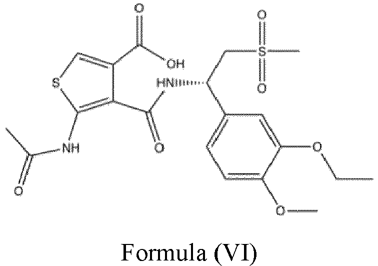

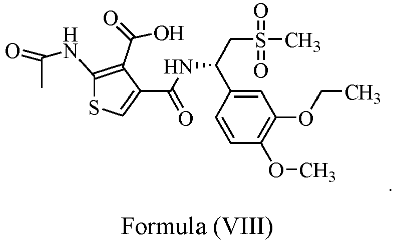

## Description

### FIELD

The present disclosure relates generally to the field of medicinal chemistry, and more particularly to the preparation of thiophene derivatives.

### BACKGROUND

Phosphodiesterase (PDE) refers to enzymes that catalyze the hydrolysis of double phosphorylated molecules in oligonucleotides, i.e., polynucleotides, which have the function of hydrolyzing intracellular second messengers (cAMP, cyclic adenosine phosphate or cGMP, guanosine phosphate), degrading intracellular cAMP or cGMP, thereby terminating the biochemical effects transmitted by these second messengers.

### SUMMARY

In one aspect, the present disclosure relates to a process for preparing a compound of formula (VII), 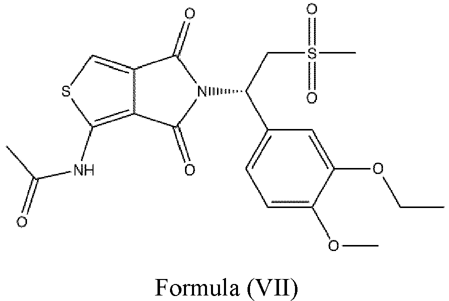 comprising subjecting a compound of formula (VI) or a compound of formula (VIII) to a condensation reaction

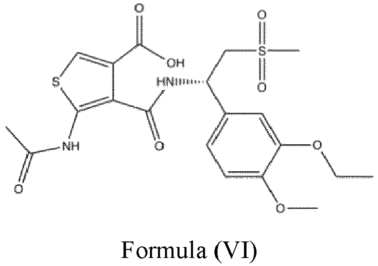

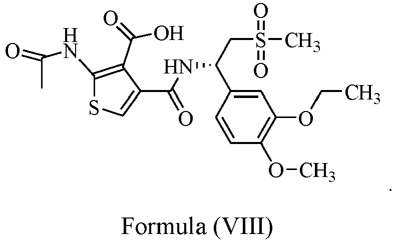

In another aspect, the present disclosure relates to a process for preparing a compound of formula (VI), 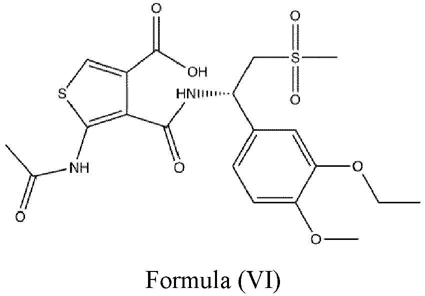 comprising subjecting a compound of formula (V) to a hydrolysis reaction

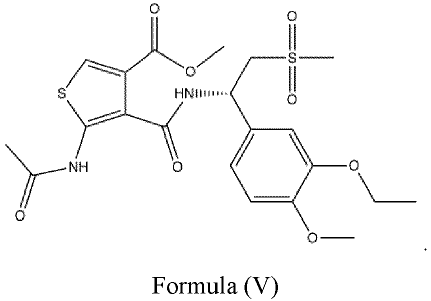

In a further aspect, the present disclosure relates to a process for preparing a compound of formula (V), 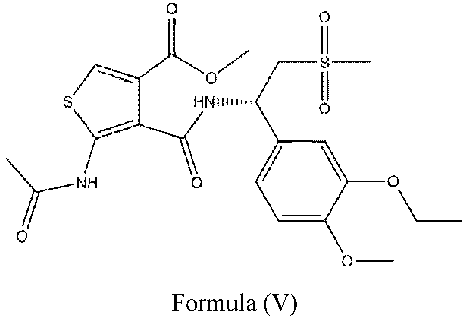 comprising subjecting a compound of formula (IV) to a condensation reaction

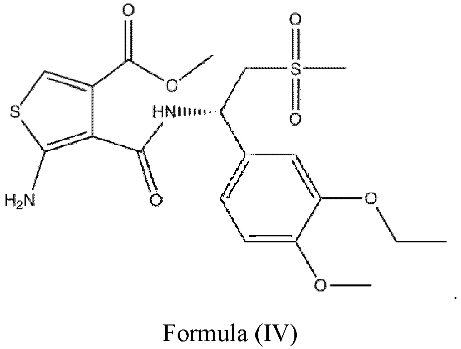

In yet another aspect, the present disclosure relates to a process for preparing a compound of formula (IV), 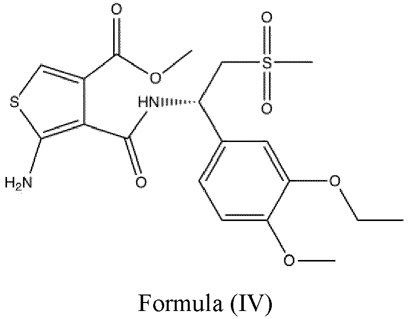 comprises subjecting a compound of formula (III) to a condensation reaction with a compound of formula (II) to obtain the compound of formula (IV)

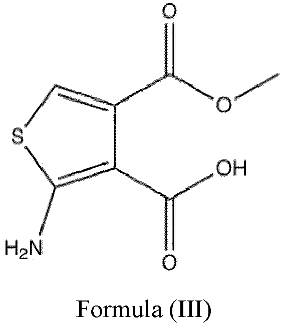

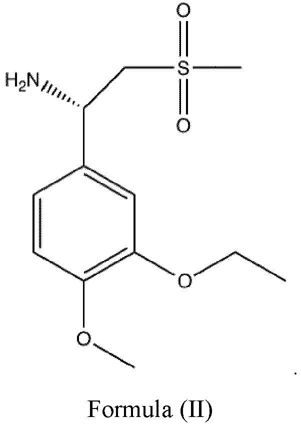

In another aspect, the present disclosure relates to a process for preparing a compound of formula (III), 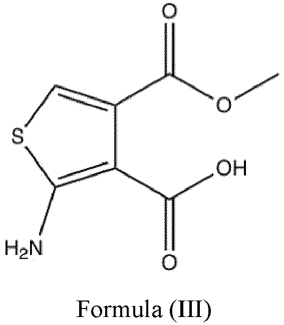 comprising reacting a compound of general formula (I) 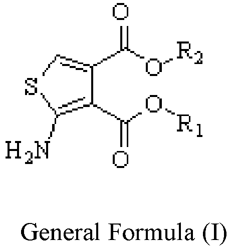 wherein R₁ ≠ R₂, R₁ and R₂ are selected from the group consisting of substituted or unsubstituted alkyl, substituted or unsubstituted alkenyl, and substituted or unsubstituted aryl.

In a further aspect, the present disclosure relates to a process for preparing a compound of formula (VII) comprising
reacting a compound of general formula (I) to obtain a compound of formula (III);
subjecting the compound of formula (III) to a condensation reaction with a compound of formula (II) to obtain a compound of formula (IV);
subjecting the compound of formula (IV) to a condensation reaction to obtain a compound of formula (V);
subjecting the compound of formula (V) to a hydrolysis reaction to obtain a compound of formula (VI); and
subjecting the compound of formula (VI) or formula (VIII) to a condensation reaction to obtain the compound of formula (VII).

In one aspect, the present disclosure relates to a process for preparing a compound of formula (VII), 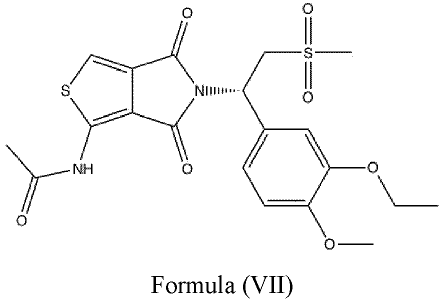 comprising subjecting a compound of formula (VIII) to a condensation reaction

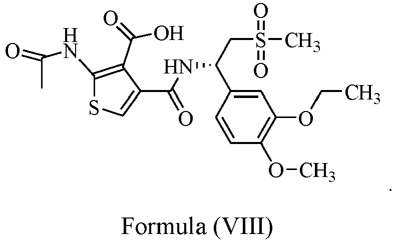

In another aspect, the present disclosure relates to a process for preparing a compound of formula (VIII), 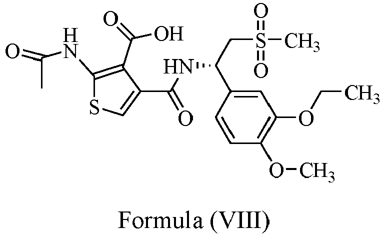 comprising subjecting a compound of formula (IX) to a hydrolysis reaction

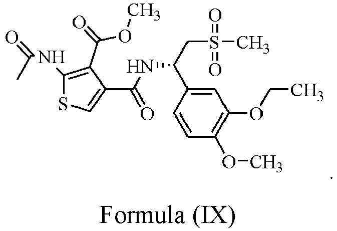

In a further aspect, the present disclosure relates to a process for preparing a compound of formula (IX), 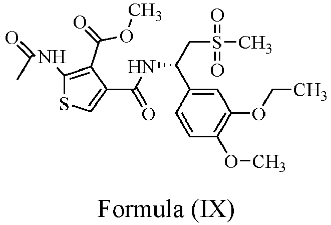 comprising subjecting a compound of formula (X) to a condensation reaction

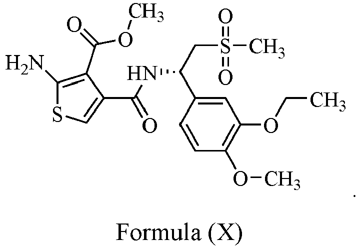

In yet another aspect, the present disclosure relates to a process for preparing a compound of formula (X), 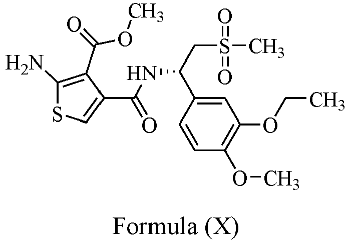 comprising subjecting a compound of formula (XI) to a condensation reaction with a compound of formula (II) to obtain the compound of formula (X),

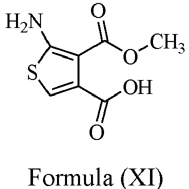

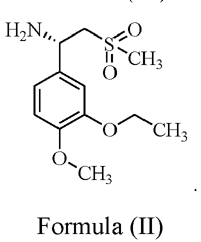

In another aspect, the present disclosure relates to a process for preparing a compound of formula (XI), 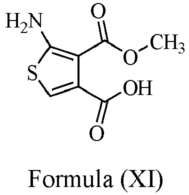 comprising reacting a compound of general formula (I) 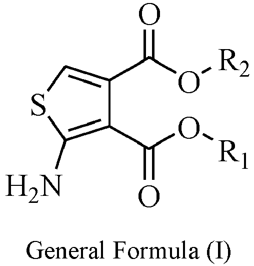 wherein R₁ ≠ R₂, R₁ and R₂ are selected from the group consisting of substituted or unsubstituted alkyl, substituted or unsubstituted alkenyl, and substituted or unsubstituted aryl.

In a further aspect, the present disclosure relates to a process for preparing a compound of Formula (VII) comprising
reacting a compound of general formula (I) to obtain a compound of formula (XI);
subjecting a compound of formula (II) to a condensation reaction with the compound of formula (XI) to obtain a compound of formula (X);
subjecting the compound of formula (X) to a condensation reaction to obtain a compound of formula (IX);
subjecting the compound of formula (IX) to a hydrolysis reaction to obtain a compound of formula (VIII); and
subjecting the compound of formula (VIII) to a condensation reaction to obtain the compound of formula (VII).

In one aspect, the present disclosure relates to a process for preparing a compound of formula (VII'), 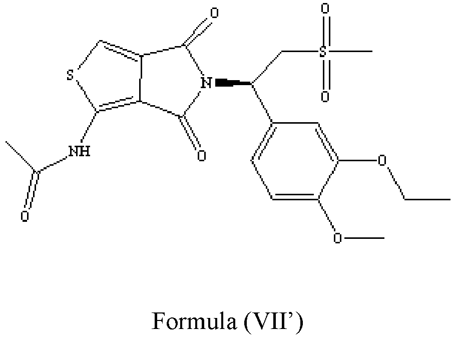 comprising subjecting a compound of formula (VI') to a condensation reaction

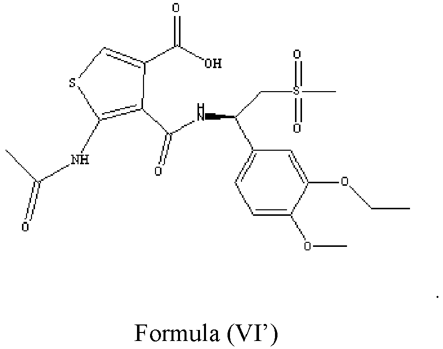

In another aspect, the present disclosure relates to a process for preparing a compound of formula (VI'), 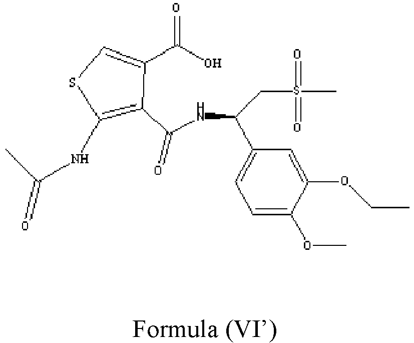 comprising subjecting a compound of formula (V') to a hydrolysis reaction

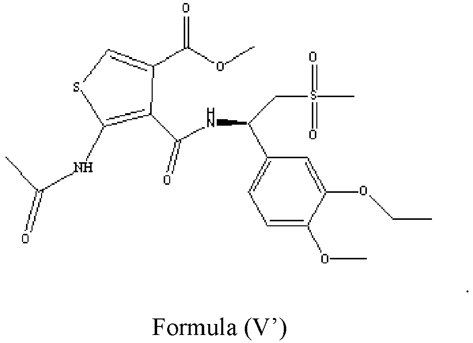

In a further aspect, the present disclosure relates to a process for preparing a compound of formula (V'), 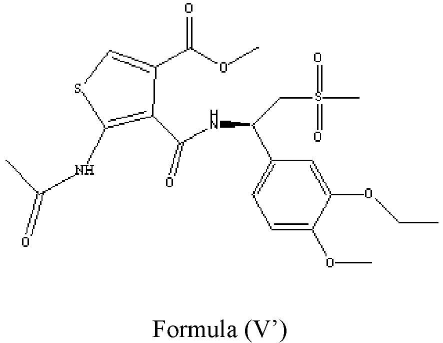 comprising subjecting a compound of formula (IV') to a condensation reaction

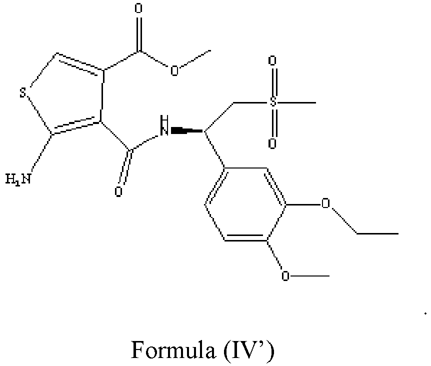

In yet another aspect, the present disclosure relates to a process for preparing a compound of formula (IV'), 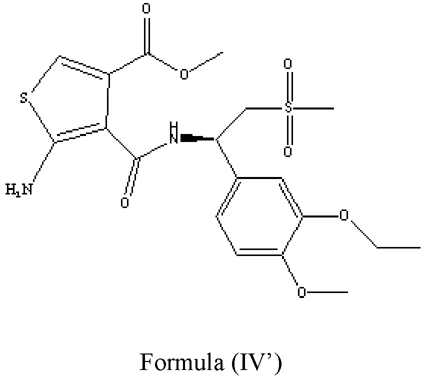 comprising subjecting a compound of formula (III) to a condensation reaction with a compound of formula (II') to obtain the compound of formula (IV')

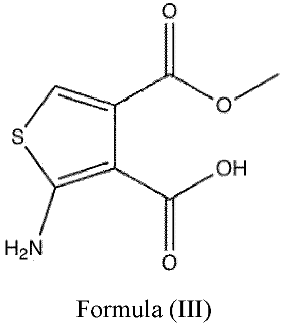

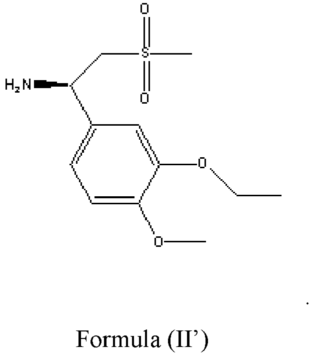

In a further aspect, the present disclosure relates to a process for preparing a compound of formula (VII') comprising
reacting a compound of general formula (I) to obtain a compound of formula (III);
subjecting the compound of formula (III) to a condensation reaction with a compound of formula (II') to obtain a compound of formula (IV');
subjecting the compound of formula (IV') to a condensation reaction to obtain a compound of formula (V');
subjecting the compound of formula (V') to a hydrolysis reaction to obtain a compound of formula (VI'); and
subjecting the compound of formula (VI') to a condensation reaction to obtain the compound of formula (VII').

In one aspect, the present disclosure relates to a process for preparing a compound of formula (VII"), 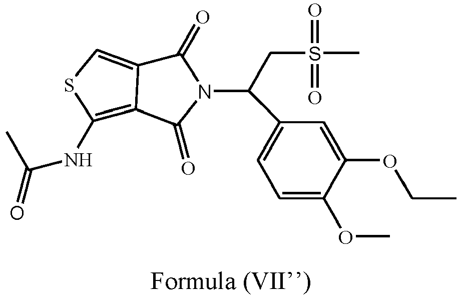 comprising subjecting a compound of formula (VI") to a condensation reaction

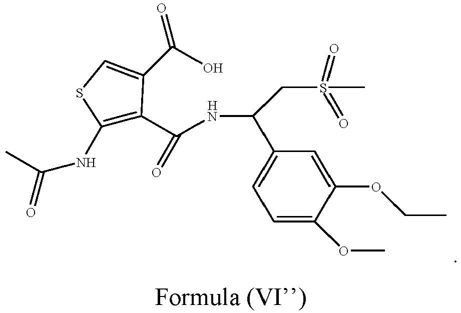

In another aspect, the present disclosure relates to a process for preparing a compound of formula (VI"), 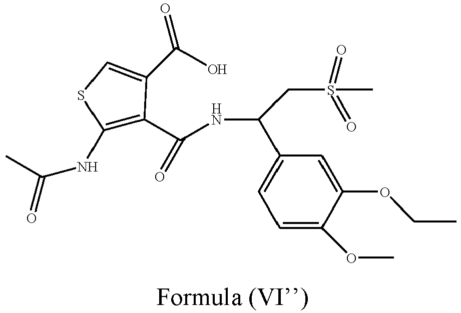 comprising subjecting a compound of formula (V") to a hydrolysis reaction

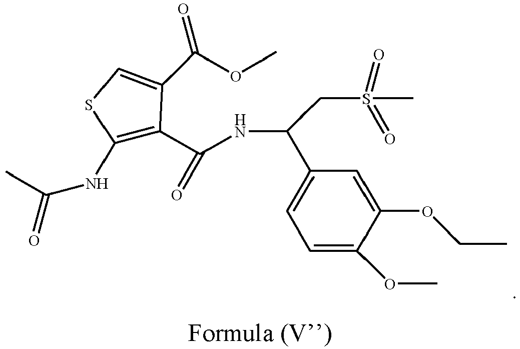

In yet another aspect, the present disclosure relates to a process for preparing a compound of formula (V"), 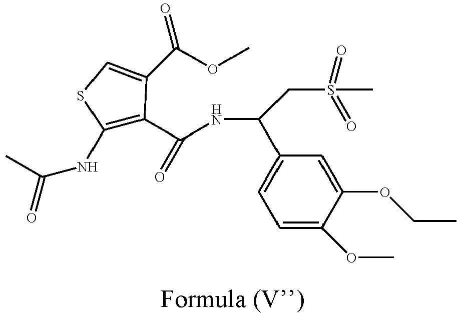 comprising subjecting a compound of formula (IV") to a condensation reaction

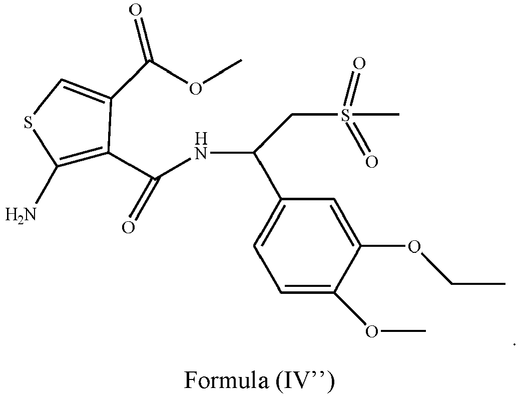

In yet another aspect, the present disclosure relates to a process for preparing a compound of Formula (IV"), 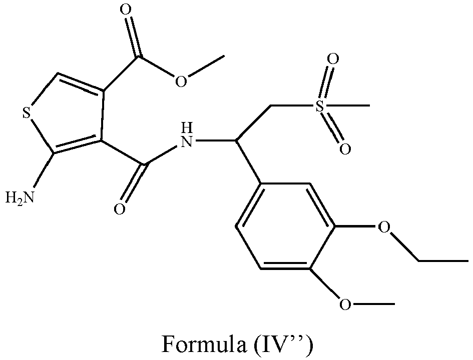 comprises subjecting a compound of formula (III) to a condensation reaction with a compound of formula (II") to obtain the compound of formula (IV")

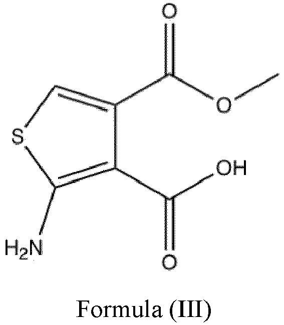

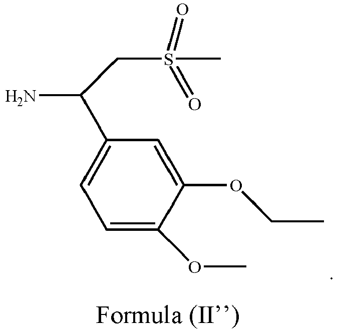

In a further aspect, the present disclosure relates to a process for preparing a compound of formula (VII") comprising
reacting a compound of general formula (I) to obtain a compound of formula (III);
subjecting a compound of formula (III) to a condensation reaction with a compound of formula (II") to obtain a compound of formula (IV");
subjecting the compound of formula (IV") to a condensation reaction to obtain a compound of formula (V");
subjecting the compound of formula (V") to a hydrolysis reaction to obtain a compound of formula (VI"); and
subjecting the compound of formula (VI") to a condensation reaction to obtain the compound of formula (VII").

### DETAILED DESCRIPTION

In the following description, certain specific details are shown to provide a thorough understanding for various disclosed embodiments. One skilled in the relevant art, however will recognize that the embodiments can be practiced without one or more these specific details, or with other methods, components materials, etc.

Unless the context required otherwise, throughout the specification and claims which follows. the term "comprise" and variations thereof, such as "comprises" and "comprising" are to be construed in an open, inclusive sense which is as "include, but not limited to".

As used in this disclosure and the appended claims, the singular reference without an indication of quantity includes the plural reference unless the context clearly dictates otherwise.

Reference throughout this specification to "one embodiment" or "an embodiment" or "in another embodiment", or "in some embodiments" means that a particular referent feature structure or characteristic described in connection with the embodiments is included in at least one embodiment. Therefore, the appearance of the phrases "in one embodiment", "in the embodiment", "in another embodiment", or "in some embodiments" in various places throughout this specification are not necessarily all referring to the same embodiment. Moreover, the particular features structures or characteristics can be combined in any suitable manner in one or more embodiments.

It should be noted that, as used in this specification and the appended claims, the singular forms "a", "an" and "the" comprise plural referents unless the context clearly stated otherwise.

### Definition

Accordingly, as used in the specification and appended claims, unless specified to the contrary, the following terms have the meaning indicated:
Certain chemical groups named herein are preceded by a shorthand notation indicating the total number of carbon atoms that are to be found in the indicated chemical group. For example, C₁-C₄alkyl describes an alkyl group, as defined below, having a total of 1 to 4 carbon atoms, and C₃-C₁₀cycloalkyl describes a cycloalkyl group having a total of 3 to 10 carbon atoms as defined below. The total number of carbon atoms in the shorthand notation does not include the carbons that can exist in the substituents of the groups described.

As used herein, the term "hydrocarbyl" refers to the aliphatic hydrocarbyl group. The hydrocarbyl moiety can be a "saturated hydrocarbyl" group, meaning that it does not contain any alkene or alkyne moieties. The hydrocarbyl moiety can also be an "unsaturated hydrocarbyl" moiety, meaning that it contains at least one alkene or alkyne moiety. An "alkene" moiety refers to a group consisting of two to eight carbon atoms and at least one carbon-carbon double bond, which is attached to the rest of the molecule by a single bond, e.g., ethenyl, prop-1-enyl, but-1-enyl, pent-1-enyl, penta-1,4-dienyl, and the like. An "alkyne" moiety refers to a straight or branched hydrocarbon chain group consisting of two to eight carbon atoms and at least one carbon-carbon triple bond, which is attached to the rest of the molecule by a single bond. The hydrocarbyl group, whether saturated or unsaturated, may be branched or linear.

The hydrocarbyl group may have one to eight carbon atoms (whenever it appears herein, a numerical range such as "one to eight" refers to each integer in the given number range; e.g. "1 to 8" means that the hydrocarbyl group may consist of one carbon atom, two carbon atoms, three carbon atoms, four carbon atoms, *etc.,* up to and including eight carbon atoms, although the present definition also covers the occurrence of term "hydrocarbyl" where no numerical range is designated).

The hydrocarbyl group may be optionally substituted, *i.e.* substituted or unsubstituted. When substituted, the substituted group(s) is(are) individually and independently selected from the group consisting of cyclohydrocarbyl, aryl, heteroaryl, heteroalicyclyl, hydroxy, hydrocarbyloxy, aryloxy, mercapto, allkylthio, arylthio, cyano, halo, carbonyl, thiocarbonyl, O-carbamyl, N-carbamyl, O-thiocarbamyl, N-thiocarbamyl, C-amido, N-amido, S-sulfinamido, N-sulfinamido, C-carboxyl, O-carboxyl, isocyanato, thiocyano, isothiocyanato, nitro, silyl, trihalomethanesulfonyl, -NR'R"(R' and R" are hydrocarbyl group defined herein) or amino comprising mono- and bi-substituted amino group, and the protected derivatives thereof. Typical hydrocarbyl groups comprise, but are not limited to, methyl, ethyl, propyl, isopropyl, butyl, isobutyl, t-butyl, pentyl, hexyl, ethenyl, propenyl, butenyl, ethynyl, propynyl and butynyl. Whenever a substituent is described as being "optionally substituted", that substituent may be substituted with one of the above substituents.

As used herein, the term "aryl" refers to a carbocycle (full carbon) or two or more fused rings (rings sharing with two adjacent carbon atoms), having completely delocalized Pi electron system. Examples of aryl group include, but are not limited to, fluorenyl, phenyl and naphthyl. The aryl group may have, for example, five to twelve carbon atoms. The aryl group of the present disclosure may be substituted or unsubstituted. Where substituted, hydrogen atom(s) is(are) substituted with one or more substituents independently selected from the group consisting of hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo, carbonyl, thiocarbonyl, O-carbamyl, N-carbamyl, O-thiocarbamyl, N-thiocarbamyl, C-amido, N-amido, S-sulfinamido, N-sulfinamido, C-carboxyl, protected C-carboxyl, O-carboxyl, isocyanato, thiocyano, isothiocyanato, nitro, silyl, trihalomethanesulfonyl, -NR'R" (R' and R" are hydrocarbyl groups defined herein) and protected amino. Whenever a substituent is described as being "optionally substituted", that substituent may be substituted with one of the above substituents.

As used herein, the term "heteroaryl" refers to a five- to eighteen-membered aromatic ring group, containing one to seventeen carbon atoms and one to ten heteroatoms selected from the group consisting of nitrogen, oxygen and sulphur. In some embodiments, the heteroaryl may be monocyclic, bicyclic, tricyclic or tetracyclic ring system, which may comprise fused or bridged ring system. Moreover, nitrogen, carbon or sulphur atom in the heteroaryl group may be optionally oxidized, and the nitrogen atom may be optionally quaternized. Exemplary examples of heteroaryl include, but are not limited to, azepinyl, acridinyl, benzimidazolyl, benzothiazolyl, benzoindolyl, benzodioxolanyl, benzofuranyl, benzoxazolyl, benzothiazolyl, benzothiadiazolyl, benzo[b][1,4]dioxepanyl, 1,4-benzodioxanyl, benzonaphthofuranyl, benzodioxolanyl, benzodioxadienyl, benzopyranyl, benzopyronyl, benzofuranyl, benzofuranonyl, benzothienyl, benzotriazolyl, benzo[4,6]imidazo[1,2-a]pyridyl, carbazolyl, cinnolinyl, dibenzofuranyl, dibenzothienyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyl, naphthyridinyl, oxadiazolyl, 2-oxoazepinyl, oxazolyl, oxiranyl, 1-phenyl-1H-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, pyrrolyl, quinazolinyl, quinoxalinyl, quinolyl, quinuclidinyl, isoquinolyl, tetrahydroquinolyl, thiazolyl, thiadiazolyl, triazolyl, tetrazolyl, triazinyl and thiophenyl. The aryl group of the present disclosure may be substituted or unsubstituted. Where substituted, hydrogen atom(s) is(are) substituted with one or more substituents independently selected from the group consisting of hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo, carbonyl, thiocarbonyl, O-carbamyl, N-carbamyl, O-thiocarbamyl, N-thiocarbamyl, C-amido, N-amido, S-sulfinamido, N-sulfinamido, C-carboxyl, protected C-carboxyl, O-carboxyl, isocyanato, thiocyano, isothiocyanato, nitro, silyl, trihalomethanesulfonyl,
- NR'R" (R' and R" are hydrocarbyl groups defined herein) and protected amino. Whenever a substituent is described as being "optionally substituted", that substituent may be substituted with one of the above substituents.

As used herein, the term "cyclohydrocarbyl" refers to stable non-aromatic monocyclic or bicyclohydrocarbyls composed of only carbon and hydrogen atoms, having three to fifteen carbon atoms, and in some embodiments having three to twelve carbon atoms, which are saturated or unsaturated and connected to the rest of the molecule through a single bond, such as cyclopropyl, cyclobutyl, cyclopentyl, cyclohexyl, cyclodecyl, *etc.* Unless stated otherwise specifically in the specification, the term "cyclohydrocarbyl" is meant to include the cyclohydrocarbyl groups as defined above, which may be optionally substituted with one or more substituents selected from the group consisting of cyclohydrocarbyl, aryl, heteroaryl, heteroalicyclyl, hydroxy, hydrocarbyloxy, aryloxy, mercapto, hydrocarbylthio, arylthio, cyano, halo, carbonyl, thiocarbonyl, O-carbamyl, N-carbamyl, O-thiocarbamyl, N-thiocarbamyl, C-amido, N-amido, S-sulfinamido, N-sulfinamido, C-carboxyl, O-carboxyl, isocyanato, thiocyanato, isothiocyanato, nitro, silyl, trihalomethanesulfonyl, -NR'R" (R' and R" are hydrocarbyl groups defined in the present disclosure) or amino comprising mono- and di-substituted amino group, and the protected derivatives thereof.

As used herein, the term "substantially complete" reaction refers to that the reactants contain more than about 80% (% yield), more preferably more than about 90% (% yield), even more preferably more than about 95% (% yield), and most preferably more than about 97% (% yield) of the desired product.

As used herein, the term "about" is used to specify that the numerical values given are approximate. For example, when referring to a reaction temperature, the term "about" means that the referred temperature includes temperatures with deviations in the range of 30%, 25%, 20%, 15%, 10%, or 5%. Likewise, the term "about" when referring to a reaction time means that the time referred to includes a time in which the deviation is in the range of 30%, 25%, 20%, 15%, 10%, or 5%.

As used herein, the term class 1 solvent refers to the class 1 solvent of ICH Q3C (R8), which is a class of solvents that should be avoided in the manufacture of pharmaceuticals. Such solvents are known human carcinogens, strongly suspected human carcinogens and environmental hazards, and more particularly refer to benzene, carbon tetrachloride, 1,2-dichloroethane, 1,1-dichloroethylene, and 1,1,1 -trichloroethane.

As used herein, the term class 2 solvent refers to the class 2 solvent of ICH Q3C (R8), which is a class of solvents that should be limited in the manufacture of pharmaceuticals. Such solvents are non-genotoxic animal carcinogens or solvents that may cause other irreversible toxicities such as neurotoxicity or teratogenicity, or have other serious but reversible toxicities, and more particularly refer to acetonitrile, chlorobenzene, chloroform, cumene, cyclohexane, cyclopentylmethyl ether, 1,2-dichloroethylene, dichloromethane, 1,2-dimethoxyethane, N,N-dimethylacetamide, N,N-dimethylformamide, 1,4-dioxane, 2-ethoxyethanol, ethylene glycol, formamide, hexane, methanol, 2-methoxyethanol, methyl butyl ketone, methyl cyclohexane, methyl isobutyl ketone, N-methyl pyrrolidone, nitromethane, pyridine, sulfolane, tert-butanol, tetrahydrofuran, tetrahydronaphthalene, toluene, 1,1,2-trichloroethylene, xylene.

As used herein, the term class 3 solvent refers to the class 3 solvent of ICH Q3C (R8), which is a class of solvents with low potential toxicity in the manufacture of pharmaceuticals. Such solvents have low potential toxicity to humans and do not require health-based exposure, and more particularly refers to acetic acid, acetone, anisole, 1-butanol, 2-butanol, butyl acetate, tert-butyl methyl ether, dimethyl sulfoxide, ethanol, ethyl acetate, diethyl ether, methyl formate, formic acid, heptane, isobutyl acetate, isopropyl acetate, methyl acetate, 3-methyl-1-butanol, methyl ethyl ketone, 2-methyl-1-propanol, 2-methyl tetrahydrofuran, pentane, 1-pentanol, 1-propanol, 2-propanol, propyl acetate, triethylamine.

If there is a discrepancy between the depicted structure and the name of the given structure, the depicted structure should be emphasized. Additionally, if the stereochemistry of a structure or portion of a structure is not indicated by, for example, a thickened or dashed line, the structure or portion of the structure should be interpreted to include all stereoisomers of the structure.

### Specific Embodiments

In one aspect, the present disclosure relates to a process for preparing a compound of Formula (VII), 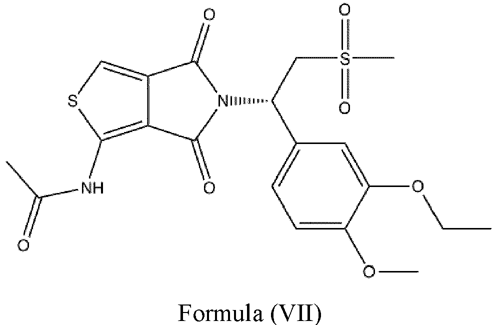 comprising subjecting a compound of formula (VI) or a compound of formula (VIII) to a condensation reaction

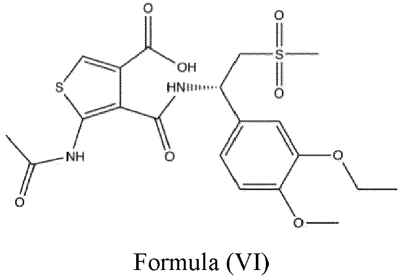

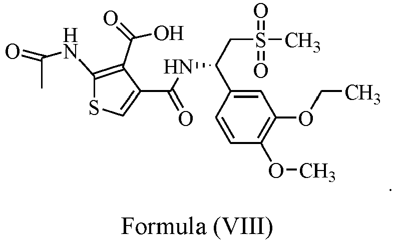

In some embodiments, exemplary examples of a reagent that can be used in the condensation reaction for preparing the compound of formula (VII) of the present disclosure include, but are not limited to, N,N'-carbonyldiimidazole (CDI), dicyclohexylcarbodiimide (DCC), 1-(3-dimethylaminopropyl)-3-ethylcarbodiimide (EDC), EDCI (EDC·HCl), dichlorosulfoxide (SOCl₂), oxalyl chloride ((COCl)₂), acetyl chloride (AcCl), acetic anhydride (Ac₂O), N,N-dimethylformamide (DMF) and any mixture thereof.

In some embodiments, exemplary examples of a reagent that can be used in the condensation reaction for preparing a compound of formula (VII) of the present disclosure include, but are not limited to, combinations of dichlorosulfoxide (SOCl₂)/N,N-dimethylformamide (DMF), oxalyl chloride ((COCl)₂)/N,N-dimethylformamide (DMF) or acetyl chloride (AcCl)/N,N-dimethylformamide (DMF).

In some embodiments, exemplary examples of a reagent that can be used in the condensation reaction for preparing a compound of formula (VII) of the present disclosure include, but are not limited to, N,N'-carbonyldiimidazole (CDI), dicyclohexylcarbodiimide (DCC), 1-(3-dimethylaminopropyl)-3-ethylcarbodiimide (EDC), EDCI (EDC·HCl), dichlorosulfoxide (SOCl₂), oxalyl chloride ((COCl)₂), acetyl chloride (AcCl), acetic anhydride (Ac₂O), N,N-dimethylformamide (DMF), dichlorosulfoxide (SOCl₂)/N,N-dimethylformamide (DMF), oxalyl chloride ((COCl)₂)/N,N-dimethylformamide (DMF) or acetyl chloride (AcCl)/N,N-dimethylformamide (DMF). The weight ratio of the above reagent to the compound of formula (VI) or the compound of formula (VIII) is about 1:1 to about 3:1.

In some embodiments, exemplary examples of a solvent that can be used in the condensation reaction for preparing the compound of formula (VII) of the present disclosure include, but are not limited to, tetrahydrofuran, dichloromethane (DCM), acetic anhydride (Ac₂O) and any mixtures thereof.

In some embodiments, the condensation reaction for preparing the compound of Formula (VII) is carried out at about -5 to about 150°C.

In some embodiments, the condensation reaction for preparing the compound of Formula (VII) is carried out at about -10 to about 40°C.

In some embodiments, the condensation reaction for preparing the compound of Formula (VII) is carried out at about 20 to about 30°C.

In some embodiments, the compound of formula (VII) is recrystallized.

In some embodiments, exemplary examples of solvents that can be used in the recrystallization of the compound of formula (VII) of the present disclosure include, but are not limited to, acetonitrile/isopropanol, ethyl acetate, acetonitrile/water, tetrahydrofuran/water and any mixtures thereof.

In another aspect, the present disclosure relates to a process for preparing a compound of Formula (VI), 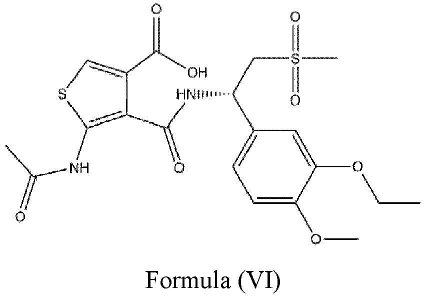 comprises subjecting a compound of formula (V) to a hydrolysis reaction

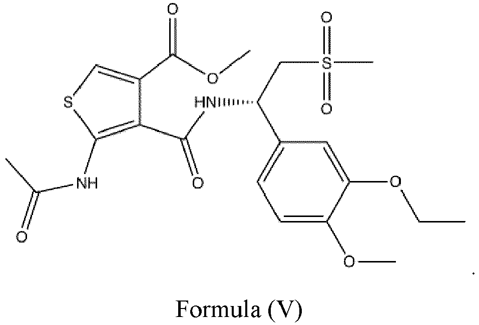

In some embodiments, the hydrolysis reaction for preparing the compound of formula (VI) is carried out under alkaline conditions.

In some embodiments, exemplary examples of salts that can be used in the hydrolysis reaction for preparing a compound of formula (VI) of the present disclosure include, but are not limited to, potassium salt, sodium salt, lithium salt and any mixtures thereof.

In some embodiments, exemplary examples of the salt that can be used in the hydrolysis reaction for preparing the compounds of formula (VI) of the present disclosure include, but are not limited to, potassium hydroxide, potassium carbonate, potassium bicarbonate, sodium hydroxide, sodium carbonate, lithium hydroxide, lithium carbonate and any mixtures thereof.

In some embodiments, the hydrolysis reaction for preparing the compound of formula (VI) of the present disclosure is carried out at about -5 to about 35°C.

In some embodiments, the hydrolysis reaction for preparing the compound of formula (VI) of the present disclosure is carried out at about 0 to about 35°C.

In some embodiments, the hydrolysis reaction for preparing the compound of formula (VI) of the present disclosure is carried out at about 20 to about 30°C.

In some embodiments, exemplary examples of a solvent that can be used in the compound of formula (VI) of the present disclosure include, but are not limited to, ethanol/water, methanol/water, isopropanol/water, acetonitrile/acetone/water, acetonitrile/acetone, ethyl acetate, tetrahydrofuran, tetrahydrofuran/water, acetone/water and any mixtures thereof.

In some embodiments, the molar ratio of the compound of formula (V) that can be used to prepare the compound of formula (VI) of the present disclosure to the salt that can be used to prepare the compound of formula (VI) in the hydrolysis reaction of the present disclosure is from about 1:1 to about 1:10.

In some embodiments, the molar ratio of the compound of formula (V) that can be used to prepare the compound of formula (VI) of the present disclosure to the salt that can be used to prepare the compound of formula (VI) in the hydrolysis reaction of the present disclosure is from about 1:1 to 1:6.

In some embodiments, the molar ratio of the compound of formula (V) that can be used to prepare the compound of formula (VI) of the present disclosure to the salt that can be used to prepare the compound of formula (VI) in the hydrolysis reaction of the present disclosure is about 1:5.

In a further aspect, the present disclosure relates to a process for preparing a compound of Formula (V), 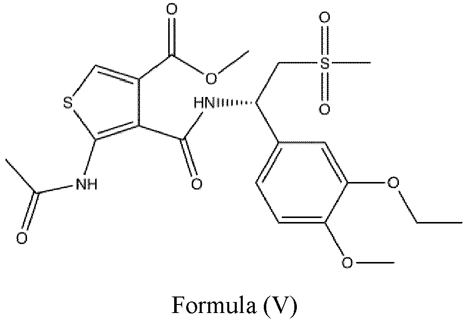 comprising subjecting a compound of formula (IV) to a condensation reaction

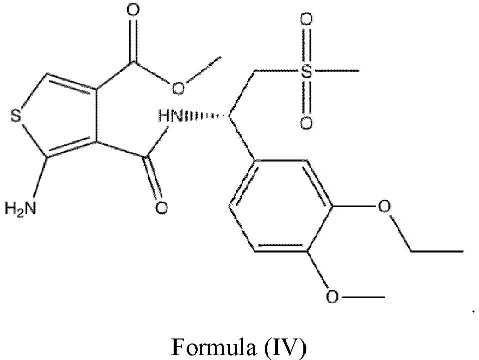

In some embodiments, exemplary examples of a reagent that can be used in the condensation reaction for preparing a compound of formula (V) of the present disclosure include, but are not limited to, acetyl chloride (AcCl), acetic anhydride (Ac₂O) and any mixture thereof.

In some embodiments, exemplary examples of a reagent that can be used in the condensation reaction for preparing a compound of formula (V) of the present disclosure include, but are not limited to, 4-dimethylaminopyridine (DMAP), N-methylmorpholine (NMM), triethylamine (TEA), pyridine (Py), N,N-diisopropylethylamine (DIEA), pyridine (Py) and any mixture thereof.

In some embodiments, exemplary examples of a reagent that can be used in the condensation reaction for preparing a compound of formula (V) of the present disclosure include, but are not limited to, Ac₂O, Ac₂O/DMAP, Ac₂O/DMAP/NMM, CH₃COCl/TEA/DCM/DMAP and CH₃COCl/Py.

In some embodiments, exemplary examples of a solvent that can be used in the condensation reaction for preparing the compound of formula (V) of the present disclosure include, but are not limited to, acetic anhydride, dichloromethane (DCM), pyridine (Py), tetrahydrofuran, acetonitrile, acetone, ethyl acetate and any mixtures thereof.

In some embodiments, the condensation reaction for preparing the compound of formula (V) of the present disclosure is carried out at about 0 to about 150°C.

In some embodiments, the condensation reaction for preparing the compound of formula (V) of the present disclosure is carried out at about 90 to about 150°C.

In some embodiments, the condensation reaction for preparing the compound of formula (V) of the present disclosure is carried out at about 125 to about 140°C.

In some embodiments, exemplary examples of a solvent that can be used to purify the compound of Formula (V) of the present disclosure include, but are not limited to, ethanol, methyl tert-butyl ether (MTBE), methanol, ethyl acetate, n-hexane, diethyl ether, tetrahydrofuran (THF) and any mixtures thereof.

In yet another aspect, the present disclosure relates to a process for preparing a compound of Formula (IV), 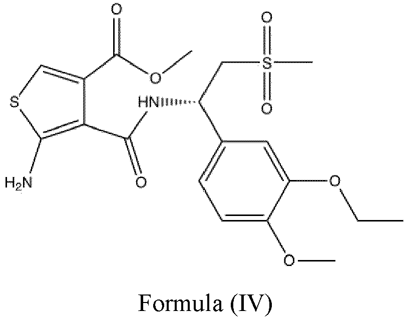 comprising subjecting a compound of formula (II) to a condensation reaction with a compound of formula (III) to obtain the compound of formula (IV)

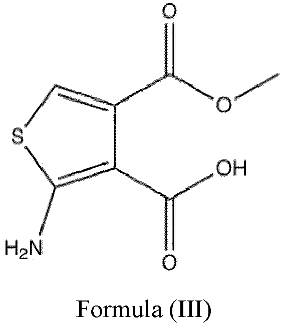

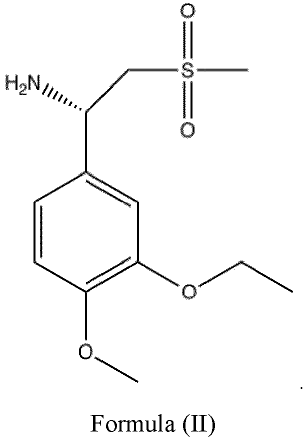

In some embodiments, exemplary examples of a condensing agent that can be used in the preparation of compound of formula (IV) of the present disclosure include, but are not limited to, carbodiimide-type condensing agents, phosphorus cationic condensing agents, urea cationic condensing agents, other suitable condensing agents and any mixtures thereof.

In some embodiments, exemplary examples of a carbodiimide-type condensing agent that can be used in the preparation of compound of formula (IV) of the present disclosure include, but are not limited to, 1-(3-dimethylaminopropyl)-3-ethylcarbodiimide (EDC), EDCI (hydrochloride salt of EDC) and dicyclohexylcarbodiimide (DCC).

In some embodiments, exemplary examples of a phosphorous cationic condensing agent that can be used in the preparation of the compound of formula (IV) of the present disclosure include, but are not limited to, 1H-benzotriazol-1-yloxytrispyrrolidinophosphonium hexafluorophosphate (PyBOP), bromotrispyrrolidinophosphonium hexafluorophosphate (PyBrOP) and benzotriazol-1-yloxytris(dimethylamino)phosphonium hexafluorophosphate (BOP).

In some embodiments, exemplary examples of a urea cationic condensing agent that can be used in the preparation of compounds of formula (IV) of the present disclosure include, but are not limited to, N,N,N',N'-tetramethyl-O-(1H-benzotriazol-1-yl)uronium hexafluorophosphate (HBTU) and 2-(7-azabenzotriazole)-N,N,N',N'-tetramethyluronium hexafluorophosphate (HATU).

In some embodiments, exemplary examples of other suitable condensing agents that can be used in the preparation of the compounds of formula (IV) of the present disclosure include, but are not limited to, 1-hydroxybenzotriazole (HOBt), 6-chloro-1-hydroxybenzotriazole (Cl-HOBt), N-hydroxy-7-azabenzotriazole (HOAt), bis(2-oxo-3-oxazolidinyl)phosphinic chloride (BOP-Cl), 3-diethoxyphosphoryloxy-1,2,3-benzotriazin-4(3H)-one (DEPBT), N-hydroxysuccinimide (HOSu), and pentafluorophenol.

In some embodiments, exemplary examples of a solvent that can be used in the preparation of compounds of formula (IV) of the present disclosure include, but are not limited to, dichloromethane, trichloromethane, acetonitrile, toluene, tetrahydrofuran, dioxane and any mixtures thereof.

In some embodiments, the condensation reaction for preparing the compound of formula (IV) of the present disclosure is carried out at about 0 to about 50°C.

In some embodiments, the condensation reaction for preparing the compound of formula (IV) of the present disclosure is carried out at about 25 to about 35°C.

In some embodiments, a weight ratio of formula (III) to formula (II) is about 1:1.3 to about 1:1.4 in the condensation reaction for preparing the compound of formula (IV) of the present disclosure.

In some embodiments, a weight ratio of formula (III) to formula (II) is about 1: 1.35 to about 1:1.4 in the condensation reaction for preparing the compound of formula (IV) of the present disclosure.

In some embodiments, a weight ratio of formula (III) to formula (II) is about 1:1.36 in the condensation reaction for preparing the compound of formula (IV) of the present disclosure.

In another aspect, the present disclosure relates to a process for preparing a compound of Formula (III), 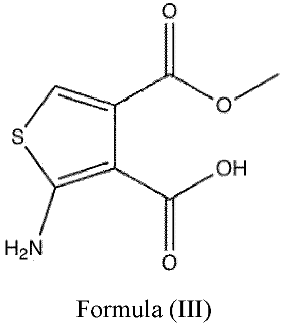 comprising reacting a compound of general formula (I) 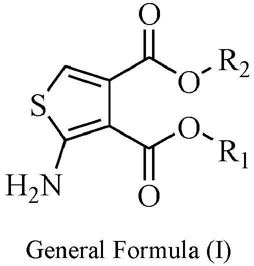 wherein R₁ ≠ R₂, R₁ and R₂ are selected from the group consisting of substituted or unsubstituted alkyl, substituted or unsubstituted alkenyl, and substituted or unsubstituted aryl.

In some embodiments, R₁ and R₂ are each independently selected from the group consisting of substituted or unsubstituted C₁-C₆alkyl, substituted or unsubstituted C₂-C₆alkenyl, and substituted or unsubstituted C₆-C₁₂aryl.

In some embodiments, R₁ and R₂ are each independently selected from the group consisting of methyl, ethyl, propyl, tert-butyl, benzyl and allyl.

In some embodiments, exemplary examples of a compound that can be used in the preparation of formula (III) of the present disclosure include, but are not limited to, trifluoroacetic acid (TFA), hydrochloric acid, hydrobromic acid, sulfonic acid, palladium on carbon, tetrakistriphenylphosphine palladium or any mixture thereof.

In some embodiments, a weight ratio of exemplary examples of trifluoroacetic acid (TFA), hydrochloric acid, hydrobromic acid, sulfonic acid, palladium on carbon, tetrakistriphenylphosphine palladium or any mixture thereof that can be used in the preparation of the compound of Formula (III) of the present disclosure to the compound of formula (I) is about 1.5:1 to about 1:1.

In some embodiments, a weight ratio of exemplary examples of trifluoroacetic acid (TFA), hydrochloric acid, hydrobromic acid, sulfonic acid, palladium on carbon, tetrakistriphenylphosphine palladium or any mixture thereof that can be used in the preparation of the compound of formula (III) of the present disclosure to the compound of formula (I) is about 1.1:1 to about 1:1.

In some embodiments, a weight ratio of exemplary examples of trifluoroacetic acid (TFA), hydrochloric acid, hydrobromic acid, sulfonic acid, palladium on carbon, tetrakistriphenylphosphine palladium or any mixture thereof that can be used in the preparation of the compound of formula (III) of the present disclosure to the compound of formula (I) is about 1.02:1.

In some embodiments, exemplary examples of a solvent that can be used in the preparation of compounds of Formula (III) of the present disclosure include, but are not limited to, dichloromethane, toluene, dichloroethane, ethyl acetate, tetrahydrofuran, 1, 4-dioxane, methanol, ethanol and any mixture thereof.

In some embodiments, the reaction of preparing the compound of formula (III) of the present disclosure is carried out at -20 to 0°C.

In some embodiments, the reaction of preparing the compound of formula (III) of the present disclosure is carried out at -15 to -5°C.

In some embodiments, exemplary examples of a compound that can be used in the preparation of Formula (III) of the present disclosure include, but are not limited to, palladium on carbon, tetrakistriphenylphosphine palladium and any mixture thereof.

In a further aspect, the present disclosure relates to a process for preparing a compound of Formula (VII) comprising
reacting a compound of general formula (I) to obtain a compound of formula (III);
subjecting the compound of formula (III) to a condensation reaction with a compound of formula (II) to obtain a compound of formula (IV);
subjecting the compound of formula (IV) to a condensation reaction to obtain a compound of formula (V);
subjecting the compound of formula (V) to a hydrolysis reaction to obtain a compound of formula (VI); and
subjecting the compound of formula (VI) to a condensation reaction to obtain the compound of formula (VII).

In one aspect, the present disclosure relates to a process for preparing a compound of Formula (VII), 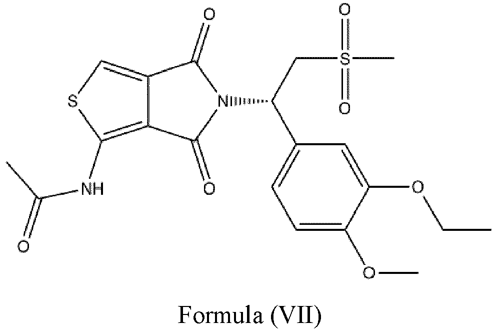 comprising subjecting a compound of formula (VIII) to a condensation reaction

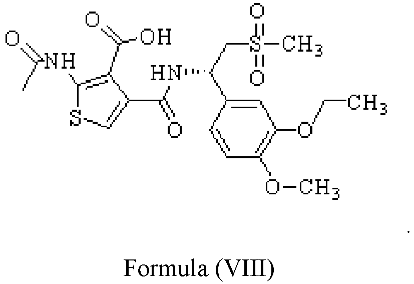

In some embodiments, exemplary examples of a reagent that can be used in the condensation reaction for preparing the compound of formula (VII) of the present disclosure include, but are not limited to, N N'-carbonyldiimidazole (CDI), dicyclohexylcarbodiimide (DCC), 1-(3-dimethylaminopropyl)-3-ethylcarbodiimide (EDC), EDCI (EDC·HCl), dichlorosulfoxide (SOCl₂), oxalyl chloride ((COCl)₂), acetyl chloride (AcCl), acetic anhydride (Ac₂O), N,N-dimethylformamide (DMF) and any mixtures thereof.

In some embodiments, the condensation reaction to prepare the compound of formula (VII) is carried out with a combination of dichlorosulfoxide (SOCl₂)/N, N-dimethylformamide (DMF), oxalyl chloride ((COCl)₂)/N,N-dimethylformamide (DMF), or acetyl chloride (AcCl)/N,N-dimethylformamide (DMF).

In some embodiments, the condensation reaction to prepare the compound of formula (VII) is carried out with a combination of dichlorosulfoxide (SOCl₂)/N,N-dimethylformamide (DMF), oxalyl chloride ((COCl)₂)/N,N-dimethylformamide (DMF), or acetyl chloride (AcCl)/N, N-dimethylformamide (DMF) in a weight ratio of the above reagent to the compound of formula (VIII) of about 1:1 to about 3: 1.

In some embodiments, exemplary examples of a solvent that can be used in the condensation reaction of the present disclosure to prepare the compound of formula (VII) include, but are not limited to, tetrahydrofuran, dichloromethane (DCM), acetic anhydride (Ac₂O) and any mixtures thereof.

In some embodiments, the condensation reaction to prepare the compound of formula (VII) is carried out at about -5 to about 150°C.

In some embodiments, the condensation reaction to prepare the compound of formula (VII) is carried out at about -10 to about 40°C.

In some embodiments, the condensation reaction to prepare the compound of formula (VII) is carried out at about 20 to about 30°C.

In some embodiments, the compound of formula (VII) is recrystallized.

In some embodiments, exemplary examples of a solvent that can be used in the recrystallization of the compound of formula (VII) of the present disclosure include, but are not limited to, acetonitrile/isopropanol, ethyl acetate, acetonitrile/water, tetrahydrofuran/water and any mixtures thereof.

In another aspect, the present disclosure relates to a process for preparing a compound of Formula (VIII), 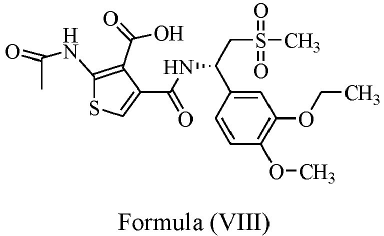 comprising subjecting a compound of formula (IX) to a hydrolysis reaction

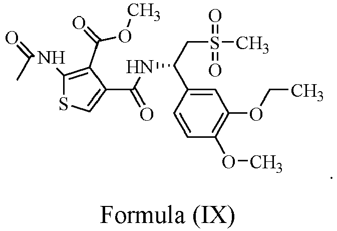

In some embodiments, the hydrolysis reaction to prepare the compound of formula (VIII) is carried out under alkaline conditions.

In some embodiments, exemplary examples of a salt that can be used in the hydrolysis reaction for preparing the compound of formula (VIII) of the present disclosure include, but are not limited to, potassium salts, sodium salts, lithium salts and any mixtures thereof.

In some embodiments, exemplary examples of a salt that can be used in the hydrolysis reaction of preparing the compound of formula (VIII) of the present disclosure include, but are not limited to, potassium hydroxide, potassium carbonate, potassium bicarbonate, sodium hydroxide, sodium carbonate, lithium hydroxide, lithium carbonate and any mixtures thereof.

In some embodiments, the hydrolysis reaction for preparing the compound of formula (VIII) of the present disclosure is carried out at about -5 to about 35°C.

In some embodiments, the hydrolysis reaction for preparing the compound of formula (VIII) of the present disclosure is carried out at about 0 to about 35°C.

In some embodiments, the hydrolysis reaction for preparing the compound of formula (VIII) of the present disclosure is carried out at about 20 to about 30°C.

In some embodiments, exemplary examples of a solvent that can be used to purify the compound of formula (VIII) of the present disclosure include, but are not limited to, ethanol/water, methanol/water, isopropanol/water, acetonitrile/acetone/water, acetonitrile/acetone, ethyl acetate, tetrahydrofuran, tetrahydrofuran/water and any mixtures thereof.

In some embodiments, the molar ratio of the compound of formula (V) that can be used in the preparation of the compound of formula (VIII) of the present disclosure to the salt that can be used in the hydrolysis reaction of the preparation of the compound of formula (VIII) of the present disclosure is from about 1:1 to about 1:10.

In some embodiments, the molar ratio of the compound of formula (V) that can be used in the preparation of the compound of formula (VIII) of the present disclosure to the salt that can be used in the hydrolysis reaction of the preparation of the compound of formula (VIII) of the present disclosure is from about 1:1 to about 1:6.

In some embodiments, the molar ratio of the compound of formula (V) that can be used to prepare the compound of formula (VIII) of the present disclosure to the salt that can be used to prepare the hydrolysis reaction of the compound of formula (VIII) of the present disclosure is about 1:5.

In a further aspect, the present disclosure relates to a process for preparing a compound of Formula (IX), 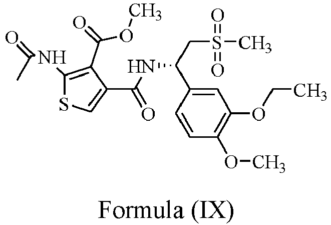 comprising subjecting a compound of formula (X) to a condensation reaction

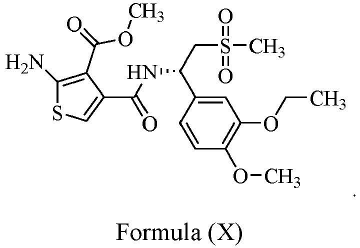

In some embodiments, exemplary examples of a reagent that can be used in the condensation reaction of the present disclosure to prepare the compound of formula (IX) include, but are not limited to, acetyl chloride (AcCl), acetic anhydride (Ac₂O) and any mixtures thereof.

In some embodiments, exemplary examples of a reagent that can be used in the condensation reaction for preparing a compound of Formula (IX) of the present disclosure include, but are not limited to, 4-dimethylaminopyridine (DMAP), N-methylmorpholine (NMM), triethylamine (TEA), pyridine (Py), N,N-diisopropylethylamine (DIEA), pyridine (Py) and any mixture thereof.

In some embodiments, exemplary examples of a reagent that can be used in the condensation reaction for preparing a compound of formula (IX) of the present disclosure include, but are not limited to, Ac₂O, Ac₂O/DMAP, Ac₂O/DMAP/NMM, CH₃COCl/TEA/DCM/DMAP and CH₃COCl/Py.

In some embodiments, exemplary examples of a solvent that can be used in the condensation reaction of the present disclosure to prepare the compound of formula (IX) include, but are not limited to, acetic anhydride, dichloromethane (DCM), pyridine (Py), tetrahydrofuran, acetonitrile, acetone, ethyl acetate and any mixtures thereof.

In some embodiments, the condensation reaction for preparing the compound of formula (IX) of the present disclosure is carried out at about 0 to about 150°C.

In some embodiments, the condensation reaction for preparing the compound of formula (IX) of the present disclosure is carried out at about 90 to about 150°C.

In some embodiments, the condensation reaction for preparing the compound of formula (IX) of the present disclosure is carried out at about 125 to about 140°C.

In some embodiments, exemplary examples of a solvent that can be used to purify compounds of formula (IX) of the present disclosure include, but are not limited to, ethanol, methyl tert-butyl ether (MTBE), methanol, ethyl acetate, n-hexane, diethyl ether, tetrahydrofuran (THF) and any mixtures thereof.

In yet another aspect, the present disclosure relates to a process for preparing a compound of Formula (X), 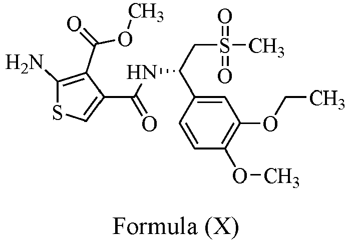 comprising subjecting a compound of formula (II) to a condensation reaction with a compound of formula (XI) to obtain the compound of formula (X)

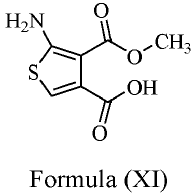

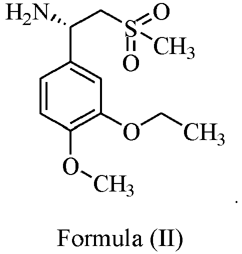

In some embodiments, exemplary examples of a condensing agent that can be used in the preparation of the compound of formula (X) of the present disclosure include, but are not limited to, carbodiimide-type condensing agents, phosphorus cationic condensing agents, urea cationic condensing agents, other suitable condensing agents and any mixtures thereof.

In some embodiments, exemplary examples of a carbodiimide-type condensing agent that can be used in the preparation of compounds of formula (X) of the present disclosure include, but are not limited to, 1-(3-dimethylaminopropyl)-3-ethylcarbodiimide (EDC), EDCI (hydrochloride salt of EDC) and dicyclohexylcarbodiimide (DCC).

In some embodiments, exemplary examples of a phosphorous cationic condensing agent that can be used in the preparation of compounds of Formula (X) of the present disclosure include, but are not limited to, 1H-benzotriazol-1-yloxytrispyrrolidinophosphonium hexafluorophosphate (PyBOP), bromotrispyrrolidinophosphonium hexafluorophosphate (PyBrOP), and benzotriazol-1-yloxytris(dimethylamino)phosphonium hexafluorophosphate (BOP).

In some embodiments, exemplary examples of a urea cationic condensing agent that can be used in the preparation of compounds of formula (X) of the present disclosure include, but are not limited to, N,N,N',N'-tetramethyl-O-(1H-benzotriazol-1-yl)uronium hexafluorophosphate (HBTU) and 2-(7-azabenzotriazole)-N,N,N',N'-tetramethyluronium hexafluorophosphate (HATU).

In some embodiments, exemplary examples of other suitable condensing agents that can be used in the preparation of the compounds of formula (X) of the present disclosure include, but are not limited to, 1-hydroxybenzotriazole (HOBt), 6-chloro-1-hydroxybenzotriazole (Cl-HOBt), N-hydroxy-7-azabenzotriazole (HOAt), bis(2-oxo-3-oxazolidinyl)phosphinic chloride (BOP-Cl), 3-diethoxyphosphoryloxy-1,2,3-benzotriazin-4(3H)-one (DEPBT), N-hydroxysuccinimide (HOSu), and pentafluorophenol.

In some embodiments, exemplary examples of a solvent that can be used in the preparation of compounds of formula (X) of the present disclosure include, but are not limited to, dichloromethane, trichloromethane, toluene, tetrahydrofuran, dioxane and any mixtures thereof.

In some embodiments, the condensation reaction to prepare the compound of formula (X) of the present disclosure is carried out at about 0 to about 50°C.

In some embodiments, the condensation reaction to prepare the compound of formula (X) of the present disclosure is carried out at about 25 to about 35°C.

In some embodiments, a weight ratio of formula (XI) to formula (II) is about 1:1.3 to about 1:1.4 in the condensation reaction for preparing the compound of formula (X) of the present disclosure.

In some embodiments, a weight ratio of formula (XI) to formula (II) is about 1: 1.35 to about 1:1.4 in the condensation reaction for preparing the compound of formula (X) of the present disclosure.

In some embodiments, a weight ratio of Formula (XI) to Formula (II) of about 1:1.36 in the condensation reaction for preparing the compound of formula (X) of the present disclosure.

In another aspect, the present disclosure relates to a process for preparing a compound of Formula (XI), 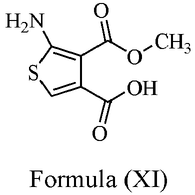 comprising reacting a compound of general formula (I) 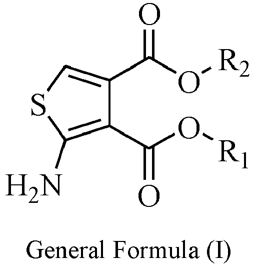 wherein R₁ ≠ R₂, R₁ and R₂ are selected from the group consisting of substituted or unsubstituted alkyl, substituted or unsubstituted alkenyl, and substituted or unsubstituted aryl.

In some embodiments, R₁ and R₂ are each independently selected from the group consisting of substituted or unsubstituted C₁-C₆alkyl, substituted or unsubstituted C₂-C₆alkenyl, and substituted or unsubstituted C₆-C₁₂aryl.

In some embodiments, R₁ and R₂ are each independently selected from the group consisting of methyl, ethyl, propyl, tert-butyl, benzyl and allyl.

In some embodiments, exemplary examples of a compound that can be used in the preparation of formula (XI) of the present disclosure include, but are not limited to, trifluoroacetic acid (TFA), hydrochloric acid, hydrobromic acid, sulfonic acid, palladium on carbon, tetrakistriphenylphosphine palladium and any mixture thereof.

In some embodiments, a weight ratio of exemplary examples of trifluoroacetic acid (TFA), hydrochloric acid, hydrobromic acid, sulfonic acid, palladium on carbon, tetrakistriphenylphosphine palladium or any mixture thereof that can be used in the preparation of the compound of formula (XI) of the present disclosure to the compound of formula (I) is about 1.5:1 to about 1:1.

In some embodiments, a weight ratio of exemplary examples of trifluoroacetic acid (TFA), hydrochloric acid, hydrobromic acid, sulfonic acid, palladium on carbon, tetrakistriphenylphosphine palladium or any mixture thereof that can be used in the preparation of compounds of formula (XI) of the present disclosure to the compound of formula (I) is about 1.1:1 to about 1:1.

In some embodiments, a weight ratio of exemplary examples of trifluoroacetic acid (TFA), hydrochloric acid, hydrobromic acid, sulfonic acid, palladium on carbon, tetrakistriphenylphosphine palladium or any mixture thereof that can be used in the preparation of the compound of formula (XI) of the present disclosure to the compound of formula (I) is about 1.02: 1.

In some embodiments, exemplary examples of a solvent that can be used in the preparation of compounds of formula (XI) of the present disclosure include, but are not limited to, dichloromethane, benzene, toluene, dichloroethane, ethyl acetate, tetrahydrofuran, 1, 4-dioxane, methanol, ethanol and any mixture thereof.

In some embodiments, the reaction of preparing a compound of formula (XI) of the present disclosure is carried out at about -20 to about 0°C.

In some embodiments, the reaction of preparing a compound of formula (XI) of the present disclosure is carried out at about -15 to about -5°C.

In some embodiments, exemplary examples of a compound that can be used in the preparation of formula (XI) of the present disclosure include, but are not limited to, palladium on carbon, tetrakistriphenylphosphine palladium and any mixture thereof.

In a further aspect, the present disclosure relates to a process for preparing a compound of Formula (VII) comprising
reacting a compound of general formula (I) to obtain a compound of formula (XI);
subjecting the compound of formula (XI) to a condensation reaction with a compound of formula (II) to obtain a compound of formula (X);
subjecting the compound of formula (X) to a condensation reaction to obtain a compound of formula (IX);
subjecting the compound of formula (IX) to a hydrolysis reaction to obtain a compound of formula (VIII); and
subjecting the compound of formula (VIII) to a condensation reaction to obtain the compound of formula (VII).

In one aspect, the present disclosure relates to a process for preparing a compound of Formula (VII'), 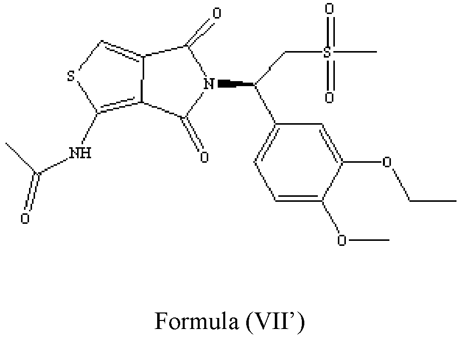 comprising subjecting a compound of formula (VI') to a condensation reaction

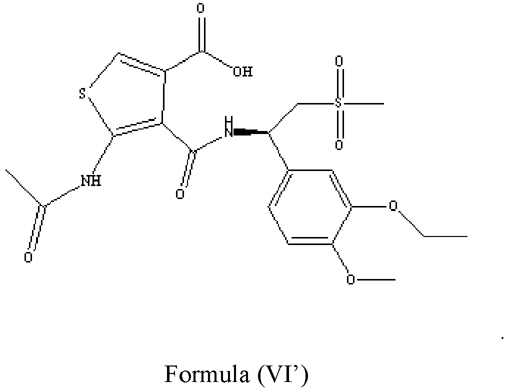

In some embodiments, exemplary examples of a reagent that can be used in the condensation reaction for preparing a compound of formula (VII') of the present disclosure include, but are not limited to, N,N'-carbonyldiimidazole (CDI), dicyclohexylcarbodiimide (DCC), 1-(3-dimethylaminopropyl)-3-ethylcarbodiimide (EDC), EDCI (EDC·HCl), dichlorosulfoxide (SOCl₂), oxalyl chloride ((COCl)₂), acetyl chloride (AcCl), acetic anhydride (Ac₂O), N,N-dimethylformamide (DMF) and any mixtures thereof.

In some embodiments, exemplary examples of a reagent that can be used in the condensation reaction for preparing the compound of Formula (VII') of the present disclosure include, but are not limited to, a combination of dichlorosulfoxide (SOCl₂)/N,N-dimethylformamide (DMF), oxalyl chloride ((COCl)₂)/N,N-dimethylformamide (DMF), or acetyl chloride (AcCl)/N,N-dimethylformamide (DMF).

In some embodiments, exemplary examples of reagent that can be used in the condensation reaction for preparing a compound of Formula (VII') of the present disclosure include, but are not limited to, N,N'-carbonyldiimidazole (CDI), dicyclohexylcarbodiimide (DCC), 1-(3-dimethylaminopropyl)-3-ethylcarbodiimide (EDC), EDCI (EDC·HCl), dichlorosulfoxide (SOCl₂), oxalyl chloride ((COCl)₂), acetyl chloride (AcCl), acetic anhydride (Ac₂O), N,N-dimethylformamide (DMF), dichlorosulfoxide (SOCl₂)/N,N-dimethylformamide (DMF), oxalyl chloride ((COCl)₂)/N,N-dimethylformamide (DMF), or acetyl chloride (AcCl)/N,N-dimethylformamide (DMF). The weight ratio of the above reagent to the compound of formula (VI') is about 1:1 to about 3:1.

In some embodiments, exemplary examples of a solvent that can be used in the condensation reaction for preparing the compound of Formula (VII') of the present disclosure include, but are not limited to, tetrahydrofuran, dichloromethane (DCM), acetic anhydride (Ac₂O) and any mixtures thereof.

In some embodiments, the condensation reaction to prepare the compound of Formula (VII') is carried out at -5 to about 150°C.

In some embodiments, the condensation reaction to prepare the compound of Formula (VII') is carried out at about -10 to about 40°C.

In some embodiments, the condensation reaction to prepare the compound of Formula (VII') is carried out at about 20 to about 30°C.

In some embodiments, the compound of formula (VII') is recrystallized.

In some embodiments, exemplary examples of a solvent that can be used in the recrystallization of a compound of Formula (VII') of the present disclosure include, but are not limited to, acetonitrile/isopropanol, ethyl acetate, acetonitrile/water, tetrahydrofuran/water and any mixtures thereof.

In another aspect, the present disclosure relates to a process for preparing a compound of Formula (VI'), 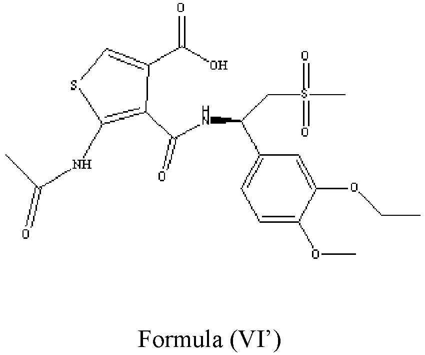 comprising subjecting a compound of formula (V') to a hydrolysis reaction

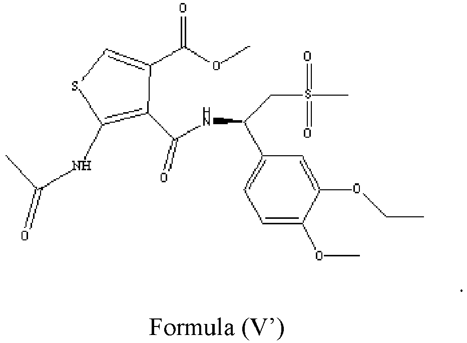

In some embodiments, the hydrolysis reaction to prepare the compound of Formula (VI') is carried out under alkaline conditions.

In some embodiments, exemplary examples of a salt that can be used in the hydrolysis reaction for preparing a compound of formula (VI') of the present disclosure include, but are not limited to, potassium salts, sodium salts, lithium salts and any mixtures thereof.

In some embodiments, exemplary examples of a salt that can be used in the hydrolysis reaction for preparing a compound of formula (VI') of the present disclosure include, but are not limited to, potassium hydroxide, potassium carbonate, potassium bicarbonate, sodium hydroxide, sodium carbonate, lithium hydroxide, lithium carbonate and any mixtures thereof.

In some embodiments, the hydrolysis reaction for preparing the compound of formula (VI') of the present disclosure is carried out at about -5 to about 35°C.

In some embodiments, the hydrolysis reaction for preparing the compound of formula (VI') of the present disclosure is carried out at about 0 to about 35°C.

In some embodiments, the hydrolysis reaction for preparing the compound of Formula (VI') of the present disclosure is carried out at about 20 to about 30°C.

In some embodiments, exemplary examples of a solvent that can be used in the compounds of Formula (VI') of the present disclosure include, but are not limited to, ethanol/water, methanol/water, isopropanol/water, acetonitrile/acetone/water, acetonitrile/acetone, ethyl acetate, tetrahydrofuran, tetrahydrofuran/water, acetone/water and any mixtures thereof.

In some embodiments, the molar ratio of the compound of formula (V') that can be used to prepare the compound of Formula (VI') of the present disclosure to the salt that can be used to prepare the hydrolysis reaction of the compound of formula (VI') of the present disclosure is from about 1:1 to about 1:10.

In some embodiments, the molar ratio of the compound of formula (V') that can be used to prepare the compound of formula (VI') of the present disclosure to the salt that can be used to prepare the hydrolysis reaction of the compound of formula (VI') of the present disclosure is from about 1:1 to about 1:6.

In some embodiments, the molar ratio of the compound of formula (V') that can be used to prepare the compound of formula (VI') of the present disclosure to the salt that can be used to prepare the hydrolysis reaction of the compound of formula (VI') of the present disclosure is about 1:5.

In a further aspect, the present disclosure relates to a process for preparing a compound of Formula (V'), 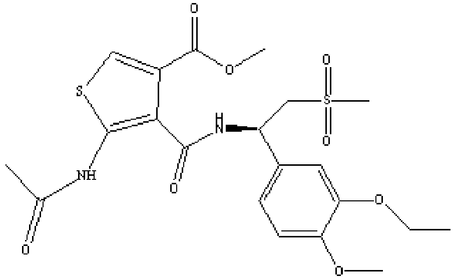 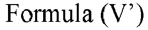 comprising subjecting a compound of formula (IV') to a condensation reaction

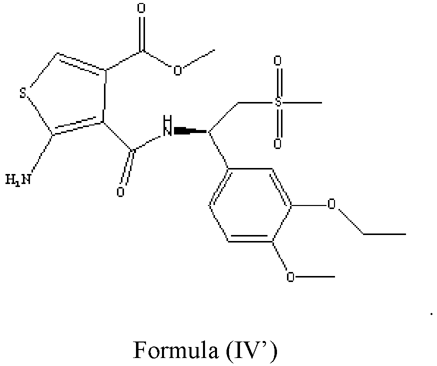

In some embodiments, exemplary examples of a reagent that can be used in the condensation reaction for preparing a compound of formula (V') of the present disclosure include, but are not limited to, acetyl chloride (AcCl), acetic anhydride (Ac₂O) and any mixtures thereof.

In some embodiments, exemplary examples of a reagent that can be used in the condensation reaction for preparing a compound of Formula (V') of the present disclosure include, but are not limited to, 4-dimethylaminopyridine (DMAP), N-methylmorpholine (NMM), triethylamine (TEA), dichloromethane (DCM), pyridine (Py), N, N-diisopropylethylamine (DIEA) and any mixtures thereof.

In some embodiments, exemplary examples of a reagent that can be used in the condensation reaction for preparing a compound of Formula (V') of the present disclosure include, but are not limited to, Ac₂O, Ac₂O/DMAP, Ac₂O/DMAP/NMM, CH₃COCl/TEA/DCM/DMAP and CH₃COCl/Py.

In some embodiments, exemplary examples of a solvent that can be used in the condensation reaction for preparing the compound of Formula (V') of the present disclosure include, but are not limited to, acetic anhydride, dichloromethane (DCM), pyridine (Py), tetrahydrofuran, acetonitrile, acetone, ethyl acetate and any mixture thereof.

In some embodiments, the condensation reaction to prepare the compound of Formula (V') of the present disclosure is carried out at about 0 to about 150°C.

In some embodiments, the condensation reaction to prepare the compound of Formula (V') of the present disclosure is carried out at about 90 to about 150°C.

In some embodiments, the condensation reaction to prepare the compound of Formula (V') of the present disclosure is carried out at about 125 to about 140°C.

In some embodiments, exemplary examples of a solvent that can be used to purify a compound of Formula (V') of the present disclosure include, but are not limited to, ethanol, methyl tert-butyl ether (MTBE), methanol, ethyl acetate, n-hexane, diethyl ether, tetrahydrofuran (THF) and any mixtures thereof.

In yet another aspect, the present disclosure relates to a process for preparing a compound of Formula (IV'), 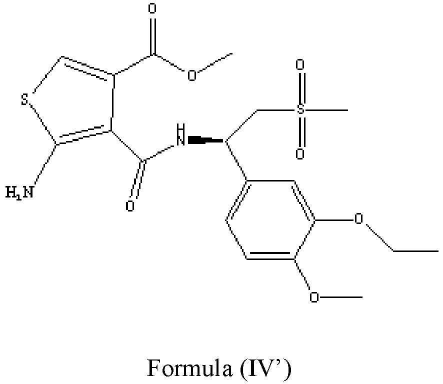 comprising subjecting a compound of formula (III) to a condensation reaction with a compound of formula (II') to obtain the compound of formula (IV')

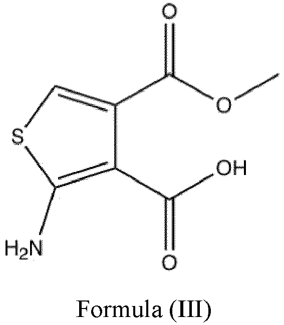

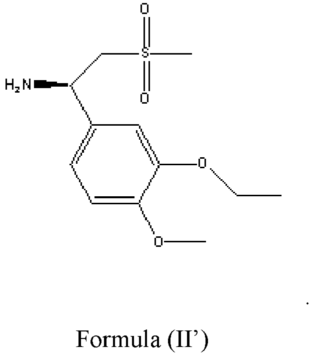

In some embodiments, exemplary examples of a condensing agent that can be used in the preparation of the compound of formula (IV') of the present disclosure include, but are not limited to, carbodiimide-type condensing agents, phosphorus cationic condensing agents, urea cationic condensing agents, other suitable condensing agents and any mixtures thereof.

In some embodiments, exemplary examples of a carbodiimide-type condensing agent that can be used in the preparation of compounds of formula (IV') of the present disclosure include, but are not limited to, 1-(3-dimethylaminopropyl)-3-ethylcarbodiimide (EDC), EDCI (hydrochloride salt of EDC) and dicyclohexylcarbodiimide (DCC).

In some embodiments, exemplary examples of a phosphorous cationic condensing agent that can be used in the preparation of compounds of formula (IV') of the present disclosure include, but are not limited to, 1H-benzotriazol-1-yloxytrispyrrolidinophosphonium hexafluorophosphate (PyBOP), bromotrispyrrolidinophosphonium hexafluorophosphate (PyBrOP), and benzotriazol-1-yloxytris(dimethylamino)phosphonium hexafluorophosphate (BOP).

In some embodiments, exemplary examples of a urea cationic condensing agent that can be used in the preparation of compounds of formula (IV') of the present disclosure include, but are not limited to, N,N,N',N'-tetramethyl-O-(lH-benzotriazol-l-yl)uronium hexafluorophosphate (HBTU) and 2-(7-azabenzotriazole)-N,N,N',N'-tetramethyluronium hexafluorophosphate (HATU).

In some embodiments, illustrative examples of other suitable condensing agents that can be used in the preparation of compounds of formula (IV') of the present disclosure include, but are not limited to, 1-hydroxybenzotriazole (HOBt), 6-chloro-1-hydroxybenzotriazole (Cl-HOBt), N-hydroxy-7-azabenzotriazole (HOAt), bis(2-oxo-3-oxazolidinyl)phosphinic chloride (BOP-Cl), 3-diethoxyphosphoryloxy-1,2,3-benzotriazin-4(3H)-one (DEPBT), N-hydroxysuccinimide (HOSu), and pentafluorophenol.

In some embodiments, exemplary examples of a solvent that can be used in the preparation of compounds of formula (IV') of the present disclosure include, but are not limited to, dichloromethane, trichloromethane, acetonitrile, toluene, tetrahydrofuran, dioxane and any mixtures thereof.

In some embodiments, the condensation reaction for preparing the compound of formula (IV') of the present disclosure is carried out at about 0 to about 50°C.

In some embodiments, the condensation reaction for preparing the compound of Formula (IV') of the present disclosure is carried out at about 25 to about 35°C.

In some embodiments, a weight ratio of formula (III) to formula (II') is about 1:1.3 to about 1:1.4 in the condensation reaction for preparing the compound of formula (IV') of the present disclosure.

In some embodiments, a weight ratio of formula (III) to formula (II') is about 1:1.35 to about 1:1.4 in the condensation reaction for preparing the compound of formula (IV') of the present disclosure.

In some embodiments, a weight ratio of formula (III) to formula (II') is about 1:1.36 in the condensation reaction for preparing the compound of formula (IV') of the present disclosure.

In a further aspect, the present disclosure relates to a process for preparing a compound of Formula (VII') comprising
reacting a compound of general formula (I) to obtain a compound of formula (III);
subjecting the compound of formula (III) to a condensation reaction with a compound of formula (II') to obtain a compound of formula (IV');
subjecting the compound of formula (IV') to a condensation reaction to obtain a compound of formula (V');
subjecting the compound of formula (V') to a hydrolysis reaction to obtain a compound of formula (VI'); and
subjecting the compound of formula (VI') to a condensation reaction to obtain the compound of formula (VII').

In one aspect, the present disclosure relates to a process for preparing a compound of Formula (VII"), 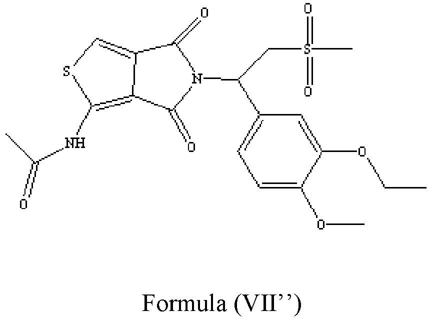 comprising subjecting a compound of formula (VI") to a condensation reaction

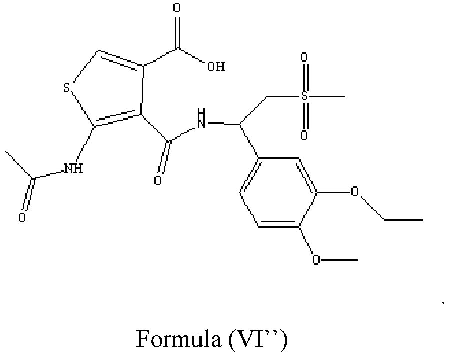

In some embodiments, exemplary examples of a reagent that can be used in the condensation reaction for preparing a compound of Formula (VII") of the present disclosure include, but are not limited to, N,N'-carbonyldiimidazole (CDI), dicyclohexylcarbodiimide (DCC), 1-(3-dimethylaminopropyl)-3-ethylcarbodiimide (EDC), EDCI (EDC·HCl), dichlorosulfoxide (SOCl₂), oxalyl chloride ((COCl)₂), acetyl chloride (AcCl), acetic anhydride (Ac₂O), N,N-dimethylformamide (DMF) and any mixtures thereof.

In some embodiments, exemplary examples of a reagent that can be used in the condensation reaction for preparing the compound of Formula (VII") of the present disclosure include, but are not limited to, a combination of dichlorosulfoxide (SOCl₂)/N,N-dimethylformamide (DMF), oxalyl chloride ((COCl)₂)/N,N-dimethylformamide (DMF), or acetyl chloride (AcCl)/N,N-dimethylformamide (DMF).

In some embodiments, exemplary examples of a reagent that can be used in the condensation reaction for preparing a compound of Formula (VII") of the present disclosure include, but are not limited to, N,N'-carbonyldiimidazole (CDI), dicyclohexylcarbodiimide (DCC), 1-(3-dimethylaminopropyl)-3-ethylcarbodiimide (EDC), EDCI (EDC·HCl), dichlorosulfoxide (SOCl₂), oxalyl chloride ((COCl)₂), acetyl chloride (AcCl), acetic anhydride (Ac₂O), N,N-dimethylformamide (DMF), dichlorosulfoxide (SOCl₂)/N,N-dimethylformamide (DMF), oxalyl chloride ((COCl)₂)/N,N-dimethylformamide (DMF), or acetyl chloride (AcCl)/N,N-dimethylformamide (DMF). The weight ratio of the above reagent to the compound of formula (VI") is about 1:1 to about 3: 1.

In some embodiments, exemplary examples of a solvent that can be used in the condensation reaction for preparing the compound of Formula (VII") of the present disclosure include, but are not limited to, tetrahydrofuran, dichloromethane (DCM), acetic anhydride (Ac₂O) and any mixtures thereof.

In some embodiments, the condensation reaction to prepare the compound of formula (VII") is carried out at about -5 to about 150°C.

In some embodiments, the condensation reaction to prepare the compound of formula (VII") is carried out at about -10 to about 40°C.

In some embodiments, the condensation reaction to prepare the compound of formula (VII") is carried out at about 20 to about 30°C.

In some embodiments, the compound of formula (VII") is recrystallized.

In some embodiments, exemplary examples of a solvent that can be used in the recrystallization of a compound of formula (VII") of the present disclosure include, but are not limited to, acetonitrile/isopropanol, ethyl acetate, acetonitrile/water, tetrahydrofuran/water and any mixtures thereof.

In another aspect, the present disclosure relates to a process for preparing a compound of Formula (VI"), 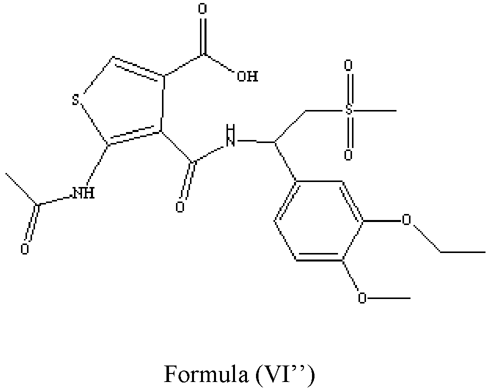 comprising subjecting a compound of formula (V") to a hydrolysis reaction

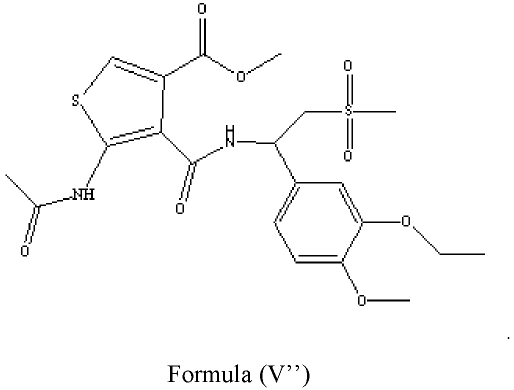

In some embodiments, the hydrolysis reaction to prepare the compound of formula (VI") is carried out under alkaline conditions.

In some embodiments, exemplary examples of a salt that can be used in the hydrolysis reaction for preparing a compound of formula (VI") of the present disclosure include, but are not limited to, potassium salts, sodium salts, lithium salts and any mixtures thereof.

In some embodiments, exemplary examples of a salt that can be used in the hydrolysis reaction for preparing a compound of formula (VI") of the present disclosure include, but are not limited to, potassium hydroxide, potassium carbonate, potassium bicarbonate, sodium hydroxide, sodium carbonate, lithium hydroxide, lithium carbonate and any mixtures thereof.

In some embodiments, the hydrolysis reaction for preparing the compound of formula (VI") of the present disclosure is carried out at about -5 to about 35°C.

In some embodiments, the hydrolysis reaction for preparing the compound of formula (VI") of the present disclosure is carried out at about 0 to about 35°C.

In some embodiments, the hydrolysis reaction for preparing the compound of Formula (VI") of the present disclosure is carried out at about 20 to about 30°C.

In some embodiments, exemplary examples of a solvent that can be used to purify a compound of Formula (VI") of the present disclosure include, but are not limited to, ethanol/water, methanol/water, isopropanol/water, acetonitrile/acetone/water, acetonitrile/acetone, ethyl acetate, tetrahydrofuran, tetrahydrofuran/water and any mixtures thereof.

In some embodiments, the molar ratio of the compound of formula (V") that can be used to prepare the compound of formula (VI") of the present disclosure to the salt that can be used to prepare the hydrolysis reaction of the compound of formula (VI") of the present disclosure is from about 1:1 to about 1:10.

In some embodiments, the molar ratio of the compound of formula (V") that can be used to prepare the compound of formula (VI"') of the present disclosure to the salt that can be used to prepare the hydrolysis reaction of the compound of formula (VI") of the present disclosure is about 1: 1 to 1:6.

In some embodiments, the molar ratio of the compound of formula (V") that can be used to prepare the compound of formula (VI") of the present disclosure to the salt that can be used to prepare the hydrolysis reaction of the compound of formula (VI") of the present disclosure is about 1:5.

In yet another aspect, the present disclosure relates to a process for preparing a compound of Formula (V"), 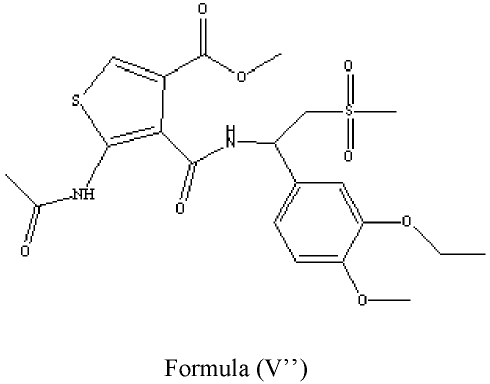 comprising subjecting a compound of formula (IV") to a condensation reaction

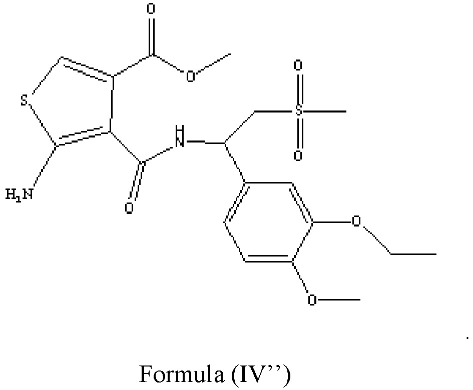

In some embodiments, exemplary examples of a reagent that can be used in the condensation reaction for preparing a compound of formula (V") of the present disclosure include, but are not limited to, acetyl chloride (AcCl), acetic anhydride (Ac₂O) and any mixtures thereof.

In some embodiments, exemplary examples of a solvent that can be used in the condensation reaction of the compounds of formula (V") of the present disclosure include, but are not limited to, 4-dimethylaminopyridine (DMAP), N-methylmorpholine (NMM), triethylamine (TEA), dichloromethane (DCM), pyridine (Py), N,N-diisopropylethylamine (DIEA) and any mixtures thereof.

In some embodiments, exemplary examples of a reagent that can be used in the condensation reaction for preparing a compound of formula (V") of the present disclosure include, but are not limited to, Ac₂O, Ac₂O/DMAP, Ac₂O/DMAP/NMM, CH₃COCl/TEA/DCM/DMAP and CH₃COCl/Py.

In some embodiments, exemplary examples of a solvent that can be used in the condensation reaction for preparing a compound of formula (V") of the present disclosure include, but are not limited to, acetic anhydride, dichloromethane (DCM), pyridine (Py), tetrahydrofuran, acetonitrile, acetone, ethyl acetate and any mixture thereof.

In some embodiments, the condensation reaction to prepare the compound of formula (V" of the present disclosure is carried out at about 0 to about 150°C.

In some embodiments, the condensation reaction to prepare the compound of formula (V") of the present disclosure is carried out at about 90 to about 150°C.

In some embodiments, the condensation reaction to prepare the compound of formula (V") of the present disclosure is carried out at about 125 to about 140°C.

In some embodiments, exemplary examples of a solvent that can be used to purify a compound of formula (V") of the present disclosure include, but are not limited to, ethanol, methyl tert-butyl ether (MTBE), methanol, ethyl acetate, n-hexane, diethyl ether, tetrahydrofuran (THF) and any mixtures thereof.

In yet another aspect, the present disclosure relates to a process for preparing a compound of Formula (IV"), 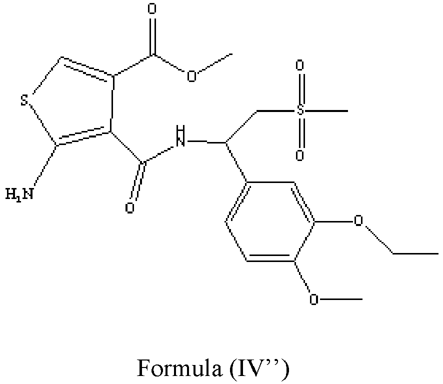 comprising subjecting a compound of formula (III) to a condensation reaction with a compound of formula (II") to obtain the compound of formula (IV")

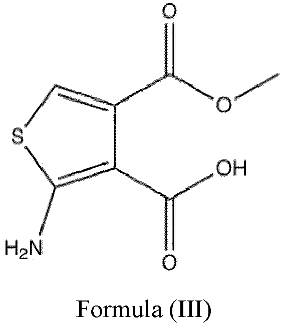

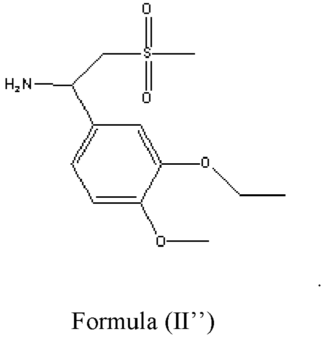

In some embodiments, exemplary examples of a condensing agent that can be used in the preparation of compounds of Formula (IV") of the present disclosure include, but are not limited to, carbodiimide-type condensing agents, phosphorus cationic condensing agents, urea cationic condensing agents, other suitable condensing agents and any mixtures thereof.

In some embodiments, exemplary examples of a carbodiimide-type condensing agent that can be used in the present disclosure include, but are not limited to, 1-(3-dimethylaminopropyl)-3-ethylcarbodiimide (EDC), EDCI (hydrochloride salt of EDC) and dicyclohexylcarbodiimide (DCC).

In some embodiments, illustrative examples of a phosphorous cationic condensing agent that can be used in the present disclosure include, but are not limited to, 1H-benzotriazol-1-yloxytrispyrrolidinophosphonium hexafluorophosphate (PyBOP), bromotrispyrrolidinophosphonium hexafluorophosphate (PyBrOP), and benzotriazol-1-yloxytris (dimethylamino) phosphonium hexafluorophosphate (BOP).

In some embodiments, exemplary examples of a urea cationic condensing agent that can be used in the present disclosure include, but are not limited to, N,N,N',N'-tetramethyl-O-(1H-benzotriazol-1-yl)uronium hexafluorophosphate (HBTU) and 2-(7-azabenzotriazole)-N,N,N',N'-tetramethyluronium hexafluorophosphate (HATU).

In some embodiments, exemplary examples of other suitable condensing agents that can be used in the present disclosure include, but are not limited to, 1-hydroxybenzotriazole (HOBt), 6-chloro-1-hydroxybenzotriazole (Cl-HOBt), N-hydroxy-7-azabenzotriazole (HOAt), bis(2-oxo-3-oxazolidinyl)phosphinic chloride (BOP-Cl), 3-diethoxyphosphoryloxy-1,2,3-benzotriazin-4(3H)-one (DEPBT), N-hydroxysuccinimide (HOSu) and pentafluorophenol.

In some embodiments, exemplary examples of a solvent that can be used in the present disclosure include, but are not limited to, dichloromethane, trichloromethane, toluene, tetrahydrofuran, dioxane and any mixtures thereof.

In some embodiments, the condensation reaction for preparing the compound of formula (IV") of the present disclosure is carried out at about 0 to about 50°C.

In some embodiments, the condensation reaction for preparing the compound of formula (IV") of the present disclosure is carried out at about 25 to about 35°C.

In some embodiments, a weight ratio of formula (III) to formula (II") is about 1:1.3 to about 1:1.4 in the condensation reactions for preparing the compound of formula (IV") of the present disclosure.

In some embodiments, a weight ratio of formula (III) to formula (II") is about 1:1.35 to about 1:1.4 in the condensation reactions for preparing the compound of formula (IV") of the present disclosure.

In some embodiments, a weight ratio of formula (III) to formula (II") is about 1:1.36 in the condensation reactions for preparing the compound of formula (IV") of the present disclosure.

In a further aspect, the present disclosure relates to a process for preparing a compound of Formula (VII") comprising
reacting a compound of general formula (I) to obtain a compound of formula (III);
subjecting the compound of formula (III) to a condensation reaction with a compound of formula (II") to obtain a compound of formula (IV");
subjecting the compound of formula (IV") to a condensation reaction to obtain a compound of formula (V");
subjecting the compound of formula (V") to a hydrolysis reaction to obtain a compound of formula (VI"); and
subjecting the compound of formula (VI") to a condensation reaction to obtain the compound of formula (VII").

The ¹H nuclear magnetic resonance (NMR) spectrum is consistent with the speculated structure. The characteristic chemical shift (δ) is one millionth of a shift from trimethylsilane to a low field, and the characteristic peaks are denoted by conventional abbreviations: for example, s denotes a singlet, dd denotes a double doublet, d denotes a doublet, t denotes a triplet, q denotes a quartet, h denotes a hexplet, m denotes a multiplet and br denotes a broad peak.

The preparation method of the present disclosure does not require chromatographic purification and is easier to scale up production. The reaction conditions of the present disclosure are easy to control, easier to scale up production, and easier to produce stability and repeatability. The preparation method of the present disclosure is more effective than the prior disclosed method. It is unexpected that the compounds directly obtained by the preparation method and route of the present disclosure without complex purification steps meet the clinical quality standard. The clinical specification refers to a substance of sufficient purity to be suitable for administration to humans. The preparation method of the present disclosure does not use a class 1 solvent and has better safety to the human body in terms of residual solvent. The preparation method of the present disclosure is economical and uses readily available raw materials. The preparation method of the present disclosure has a better single-step yield and also a better overall yield.

Hereinafter, the present disclosure is explained in detail through the following examples in order to better understand various aspects and advantages of the present disclosure. However, it should be understood that the following examples are non-limiting and are only used to illustrate some embodiments of the present disclosure. One skilled in the art may carry out the solutions disclosed herein with appropriate adjustments according to the reaction equipment, the scale of the reaction equipment, the principles of the reaction equipment, and the like.

### Examples

### Abbreviations:

| | |
|---|---|
| CDI | N,N'-carbonyl diimidazole |
| HPLC | High performance liquid chromatography |
| DMF | N,N-dimethylformamide |
| Cl-HOBt | 6-chloro-1-hydroxybenzotriazole |
| EDC·HCl | 1-ethyl-3-(3-dimethylaminopropyl) carbodiimide hydrochloride |
| TLC | Thin layer chromatography |

### Example 1

### Preparation of (S)-N-[5-[1-(3-ethoxy-4-methoxyphenyl)-2-(methylsulfonyl)ethyl]-4 ,6-dioxo-5,6-dih ydro-4H-thieno[3,4-c]pyrrol-1-yl]acetamide

To a 100 mL single-necked flask, were added (S)-5-acetamido -4-[[1-(3-ethoxy-4-methoxyphenyl)-2-(methylsulfonyl)ethyl]aminocarbonyl]thiophen e-3-carboxylic acid (5 g, 10.3mmol) and tetrahydrofuran (25 mL). The mixture was dissolved with stirring at room temperature (25°C). CDI (5 g, 31.0mol) was added portionwise to the single-necked flask. The mixture was stirred at room temperature for 2 to 5 hours, monitored by HPLC. The reaction was stopped and quenched with water. The mixture was concentrated in vacuo to give a concentrate. The concentrate was dissolved in dichloromethane, washed with water and brine, and dried over anhydrous sodium sulfate. The solvent was removed to give a residue. The residue was added to acetonitrile (5 mL) and heated to reflux until clear. Isopropanol (30 mL) was added to reflux for 2 hours. The heating was stopped. The resultant was cooled to the room temperature with stirring. The resultant was filtered with pump to give a filter cake. The filter cake was dried in vacuo to give 3.7g of the title compound (purity 99.32%, yield: 77.1%). ¹H NMR (500MHz, DMSO-d₆) δ 11.71 (s, 1H), 7.73 (s, 1H), 7.04 (s, 1H), 6.91-6.94 (m, 2H), 5.71 (dd, 1H), 4.33 (dd, 1H), 4.09 (dd, 1H), 3.99 (q, 2H), 3.72 (s, 3H), 3.00 (s, 3H), 2.23 (s, 3H), 1.31 (t, 3H).¹³C NMR (100 MHz, DMSO-d6) δ 170.03, 162.69, 162.30, 149.53, 148.54, 141.70, 131.45, 130.60, 120.42, 120.23, 115.00, 15.39., 112.47, 23.07, 56.20, 53.74, 47.99, 41.74, 23.07, 15.39.LCMS: 489.1 ([M+Na]⁺), 465.0 ([M-H]⁻). Elemental Analysis Results: [C₂₀H₂₂N₂O₇S₂]: C, 50.78; H, 4.80; N, 5.93; Found: C, 50.33; H, 4.70; N, 5.69.

### Example 2

### Preparation of (S)-N-[5-[1-(3-ethoxy-4-methoxyphenyl)-2-(methylsulfonyl)ethyl]-4,6-dioxo-5,6-dih ydro-4H-thieno[3,4-c]pyrrol-1-yl]acetamide

To a reaction kettle were added (S)-5-acetamido-4-[[1-(3-ethoxy-4-methoxyphenyl)-2-(methylsulfonyl)ethyl]aminoca rbonyl]thiophene-3-carboxylic acid (4.47 g) and tetrahydrofuran (23.5 mL). The mixture was dissolved with stirring at room temperature. CDI (7.83g) was added portionwise to the reaction vessel. The reaction was carried out at 25°C for 6 hours and was stopped by HPLC monitoring and quenched with water. The mixture was concentrated in vacuo to give an oil. The oil was dissolved in dichloromethane, washed with water and brine, and dried over anhydrous sodium sulfate. The solvent was removed to give a residue. The residue was added to acetonitrile (15 mL) and heated to reflux until clear. Isopropanol (90 mL) was added to reflux for 2 hours. The heating was stopped. The resultant was stirred at room temperature overnight and was filtered by pump to give a filter cake. The filter cake was dried in vacuo to give 2.58 g of the title compound (yield: 60%, purity: 98.3%).

### Example 3

### Preparation of (S)-N-[5-[1-(3-ethoxy-4-methoxyphenyl)-2-(methylsulfonyl)ethyl]-4,6-dioxo-5,6-dih ydro-4H-thieno[3,4-c] pyrrol-1-yl]acetamide

To a 50 mL single-necked flask were added (S)-5-acetamido-4-[[1-(3-ethoxy-4-methoxyphenyl)-2-(methylsulfonyl)ethyl]aminoca rbonyl]thiophene-3-carboxylic acid (5 g) and acetic anhydride (15 mL). The mixture was heated to reflux for 2 to 3 hours and monitored by HPLC until the reaction was substantially completed. After cooling to room temperature, the reaction was diluted with dichloromethane, washed with water and brine, and dried over anhydrous sodium sulfate. The solvent was removed to give a residue. The residue was added to acetonitrile (5 ml) and heated to reflux until clear. Isopropanol (30 mL) was added and refluxed for 2 hours. The heating was stopped. The resultant was stirred at room temperature and filtered by pump to give a filter cake. The filter cake was dried in vacuo to give 3.4 g of the title compound (purity 89%, yield: 71%). ¹H NMR (600MHz, CDCl₃-d₁) δ 9.39 (s, 1H), 7.33 (s, 1H), 7.07-7.09 (m, 2H), 6.84 (d, 1H), 5.83 (dd, 1H), 4.57 (dd, 1H), 4.11 (q, 2H), 3.86 (s, 3H), 3.76 (dd, 1H), 2.89 (s, 3H), 2.30 (s, 3H), 1.47 (t, 3H). LCMS: 489.3 ([M+Na]⁺), 465.4 ([M-H]⁻).

### Example 4

### Preparation of (S)-5-acetamido-4-[[1-(3-ethoxy-4-methoxyphenyl)-2-(methylsulfonyl)ethyl]aminoca rbonyl]thiophene-3-carboxylic acid

To a 500 mL three-necked flask were added (S)-methyl-5-acetamido-4-[[1-(3-ethoxy-4-methoxyphenyl)-2-(methylsulfonyl)ethyl]a minocarbonyl]thiophene-3-carboxylate (16 g, 0.0321 mol) and ethanol (160mL). The mixture was dissolved with stirring and cooled to below 0°C. An aqueous solution of 160 mL 4.2% lithium hydroxide was added dropwise. The temperature in the three-necked bottle was controlled to be less than 0°C during the addition. After the addition was completed, the reaction was allowed to warm to room temperature, stirred for 3 to 6 hours, and monitored by HPLC until the reaction was essentially completed. After completion of the reaction, 4 mol/L hydrochloric acid was added dropwise to adjust the pH to 5 to 6. The mixture was concentrated in vacuo to give an oil. The oil was dissolved in water. 4mol/L hydrochloric acid was added dropwise to adjust the pH to 1 to 2. To the mixture were added acetonitrile (120 mL) and acetone (40 mL). The resultant was stirred for 3 hours, filtered by pump to obtain a filter cake. The filter cake was dried in vacuo to obtain 11.3 g of the title compound (purity 98%, yield: 73%). ¹H NMR (600MHz, DMSO-d₆) δ 11.41 (s, 1H), 10.09 (d, 1H), 7.85 (s, 1H), 7.09 (d, 1H), 6.98 (dd, 1H), 6.93 (d, 1H), 5.55 (dd, 1H), 4.05 (q, 2H), 3.80 (dd, 1H), 3.75 (s, 3H), 3.65 (dd, 1H), 2.90 (s, 3H), 2.19 (s, 3H), 1.34 (t, 3H). LCMS: 507.4 ([M+Na]⁺), 483.4 ([M-H]⁻)

### Example 5

### Preparation of (S)-2-methyl-5-acetamido-4-[[1-(3-ethoxy-4-methoxyphenyl)-2-(methylsulfonyl)ethyl ]aminocarbonyl]thiophene-3-carboxylate

To a 250 mL three-necked flask were added (S)-methyl-5-amino-4-[[1-(3-ethoxy-4-methoxyphenyl)-2-(methylsulfonyl)ethyl]amin ocarbonyl]thiophene-3-carboxylate (20 g) and acetic anhydride (70 mL). The mixture was heated to reflux until clear and stirred for 0.5 hour. After cooling to 80 to 90°C, the reaction mixture was stirred with ethanol (210 mL) to precipitate a solid. The solid was further stirred at room temperature and filtered by pump to give a filter cake. The filter cake was dried in vacuo to give 17.8g of the title compound (yield: 82%, purity: 99%). ¹H NMR (600MHz, CDCl₃-d₁) δ 12.97 (s, 1H), 11.04 (d, 1H), 7.85 (s, 1H), 7.03-7.04 (m, 2H), 6.91 (d, 1H), 5.67 (dd, 1H), 4.18 (q, 2H),3.93 (S, 3H), 3.89 (s, 3H), 3.74 (dd, 1H), 3.49 (dd, 1H), 2.70 (s, 3H), 2.29 (s, 3H), 1.50 (t, 3H). LCMS: 521.3 ([M+Na]⁺), 497.5 ([M-H]⁻).

### Example 6

### Preparation of (S)-2-methyl-5-acetamido-4-[[1-(3-ethoxy-4-methoxyphenyl)-2-(methylsulfonyl)ethyl ]aminocarbonyl]thiophene-3-carboxylate

To a reaction kettle were added (S)-methyl-5-amino-4-[[1-(3-ethoxy-4-methoxyphenyl)-2-(methylsulfonyl)ethyl]amin ocarbonyl]thiophene-3-carboxylate (3.63 g) and acetic anhydride (12.7 mL).The mixture was heated to 125 to 140°C and stirred for 0.5 hour. After cooling to 80 to 90°C, the reaction mixture was stirred with ethanol (38 mL) to precipitate a solid. The solid was further stirred at room temperature for 5 hours and filtered by pump to give a filter cake. The filter cake was dried in vacuo to give 3.52g of the title compound (yield: 89%, purity 99.50%).

### Example 7

### Preparation of (S)-2-methyl-5-acetamido-4-[[1-(3-ethoxy-4-methoxyphenyl)-2-(methylsulfonyl)ethyl ]aminocarbonyl]thiophene-3-carboxylate

To a 100 mL one-necked flask were added (S)-methyl-5-amino-4-[[1-(3-ethoxy-4-methoxyphenyl)-2-(methylsulfonyl)ethyl]amin ocarbonyl]thiophene-3-carboxylate (12 g, 0.0263 mol), acetyl chloride (2.5 g, 0.0318 mol) and pyridine (30 mL). The mixture was stirred at room temperature overnight. The mixture was concentrated in vacuo to give a concentrate. The concentrate was dissolved in dichloromethane, washed with 2mol/L hydrochloric acid, water and brine, and dried over anhydrous sodium sulfate. The solvent was removed to give a residue. The residue was purified to give 10.1g of the title compound (yield: 77.1%, purity: 96%). ¹H NMR (600MHz, CDCl₃-d₁) δ12.98 (s, 1H), 11.05 (d, 1H), 7.86 (s, 1H), 7.03-7.05 (m, 2H), 6.91 (d, 1H), 5.67 (dd, 1H), 4.17 (t, 2H), 3.93 (S, 3H), 3.89 (s, 3H), 3.74 (dd, 1H), 3.49 (dd, 1H), 2.70 (s, 3H), 2.29 (s, 3H), 1.51 (t, 3H). LCMS: 521.3 ([M+Na]⁺), 497.5 ([M-H]⁻).

### Example 8

### Preparation of (S)-methyl-5-amino-4-[[1-(3-ethoxy-4-methoxyphenyl)-2-(methylsulfonyl)ethyl]amin ocarbonyl]thiophene-3-carboxylate

To a 500 mL three-necked bottle were added 2-amino-4-(methoxycarbonyl) thiophene-3-carboxylic acid (25 g, 0.124 mol), (S)-1-(3-ethoxy-4-methoxyphenyl)-2-methylsulfonylethylamine (34 g, 0.125 mol) (prepared in accordance with the patent CN104245668B), Cl-HOBt (25.3 g, 0.150 mol) and dichloromethane (300 mL). The mixture was stirred for 10 to 20 minutes. EDC·HCl (28.5g, 0.149mol) was added to a three-necked bottle. After the addition was completed, the reaction was stopped after heating to an internal temperature of 25 to 35°C for 3 to 6 hours. After completion of the reaction, 1 mol/L hydrochloric acid was added to a three-necked flask. A solid was precipitated and filtered by pump. The filtrate was separated from the organic layer. The organic layer was washed with water and brine, and dried over anhydrous sodium sulfate. The solvent was removed and the residue was added to ethyl acetate (250 mL). The mixture was heated to reflux for 2 hours. The heating was stopped. The resultant was allowed to cool to room temperature and was filtered by pump to give a filter cake. The filter cake was dried in vacuo to give 36.2g of the title compound (yield: 64%, purity: 99%). ¹H NMR (500MHz, DMSO-d₆) δ 9.31 (d, 1H), 7.31 (s, 1H), 7.26 (s, 2H), 36.2/7.06 (s, 1H), 6.92 (s, 2H), 5.45 (dd, 1H), 4.05 (q, 2H), 3.73 (s, 3H), 3.69 (s, 3H), 3.57-3.66 (m, 2H), 2.79 (s, 3H), 1.33 (t, 3H). ¹³C NMR (100MHz, DMSO-d₆) δ 165.12, 164.63, 162.16, 149.03, 15.42., 133.52, 128.90, 119.81, 119.79, 112.45, 112.29, 106.52, 64.34, 59.53, 56.15, 52.94, 48.47, 41.94, 15.42. LCMS: 457.1 ([M+H]⁺), 455.0 ([M-H]⁻).

### Example 9

### Preparation of (S)-methyl-5-amino-4-[[1-(3-ethoxy-4-methoxyphenyl)-2-(methylsulfonyl)ethyl]amin ocarbonyl]thiophene-3-carboxylate

2-amino-4-(methoxycarbonyl)thiophene-3-carboxylic acid (7.5 g), (S)-1-(3-ethoxy-4-methoxyphenyl)-2-methylsulfonylethylamine (9.3 g), Cl-HOBt (7.6 g) and dichloromethane (102 mL) were stirred for 10 to 20 minutes. EDC·HCl (8.6 g) was added to the reaction solution. After completion of the addition, the reaction was heated to an internal temperature of 25 to 35°C. The reaction was monitored by HPLC for 3 to 6 hours. After completion of the reaction, 1 mol/L hydrochloric acid (1.6 g) was added to the reaction vessel. A solid was precipitated. The organic layer was filtered by pump. The filtrate was separated, washed with water and brine and dried over anhydrous sodium sulfate. The solvent was removed and the residue was added to ethyl acetate (6.7g) and heated to reflux for 2 hours. The heating was stopped. The resultant was allowed to cool to room temperature and filtered by pump to give a filter cake. The filter cake was dried in vacuo to give 14.6g of the title compound (yield: 60%, purity: 97%).

Alternatively, (S)-methyl-5-amino-4-[[1-(3-ethoxy-4-methoxyphenyl)-2-(methylsulfonyl)ethyl]amin ocarbonyl]thiophene-3-carboxylate was prepared similarly according to the method in Examples 12 or 13 as described above, except that N-hydroxysuccinimide (HOSu), N-hydroxy-7-azobenzotriazole (HOAt) or HOBt was used in place of Cl-HOBt to obtain a similar reaction effect.

Alternatively, (S)-methyl-5-amino-4-[[1-(3-ethoxy-4-methoxyphenyl)-2-(methylsulfonyl)ethyl]amin ocarbonyl]thiophene-3-carboxylate was prepared similarly according to the method in Example 12 or 13 as described above, except that tetrahydrofuran, N,N-dimethylformamide or trichloromethane was used instead of dichloromethane to obtain a similar reaction effect.

Alternatively, (S)-methyl-5-amino-4-[[1-(3-ethoxy-4-methoxyphenyl)-2-(methylsulfonyl)ethyl]amin ocarbonyl]thiophene-3-carboxylate was prepared similarly according to the method in Examples 12 or 13 as described above, except that the reaction temperature was from about 15°C to about 35°C to obtain a similar reaction effect.

### Example 10

### Preparation of 2-amino-4-(methoxycarbonyl)thiophene-3-carboxylic acid

To a 250 mL three-necked bottle were added 3-tert-butyl-4-methyl-2-aminothiophene-3,4-dicarboxylate (47 g, 0.183 mol, prepared by the method disclosed in Organic Letters, 13 (1), 38-41; 2011) and dichloromethane (60 mL). The mixture was dissolved with stirring and cooled to -10°C. Trifluoroacetic acid (48 g, 0.421 mol) was added to dichloromethane (30 mL) to prepare solution A. The solution A was added dropwise to the three-necked bottle. The temperature in the three-necked bottle was controlled below -5°C during the dropwise addition. After the addition was completed, the internal temperature is controlled to be less than 0°C for 10 to 30 minutes. The reaction mixture was stirred with methyl tert-butyl ether (300 ml) for 2 hours, filtered by pump to obtain a filter cake. The filter cake was dried in vacuo to obtain 28.2g of the title compound (yield: 77%, purity: 97.4%). ¹H NMR (600MHz, Acetone-d₆) δ 7.75 (s, 1H), 7.52 (s, H), 3.96 (s, 3H). LCMS: 202.1 ([M+H]⁺).

### Example 11

### Preparation of 2-amino-4-(methoxycarbonyl)thiophene-3-carboxylic acid

3-tert-butyl-4-methyl-2-aminothiophene-3,4-dicarboxylate (5.5 g) and dichloromethane (9.6 g) were dissolved with stirring and cooled to -10°C. The resulting solution was designated A. Trifluoroacetic acid (5.6 g) was added to dichloromethane (4.8 g) to prepare solution B. The solution B is cooled to 0°C or less. The solution B was added dropwise to the solution A. During the dropwise addition, the temperature was controlled to be lower than -10±5°C. After the addition was completed, the reaction was carried out for 10 to 30 minutes. After completion of the reaction, the reaction solution was added to methyl tert-butyl ether (78.3 g) and stirred for 2 hours, filtered by pump to obtain a filter cake. The filter cake was dried in vacuo to obtain 3.7g of the title compound (yield: 86%, sample purity: 97.4%).

### Example 12 Preparation of 2-amino-4-(methoxycarbonyl)thiophene-3-carboxylic acid

3-Benzyl-4-methyl-2-aminothiophene-3,4-dicarboxylate (50 g, 0.172 mol, prepared by the method disclosed in Organic Letters, 13 (1), 38-41; 2011) was added to ethyl acetate (900ml). The mixture was stirred to dissolve, which was denoted as solution A. After inerting a 2 L hydrogenation flask with nitrogen, 10% wet Pd/C (12.5 g) was added. Ethyl acetate (100 mL) was added to the hydrogenation flask to submerge the catalyst. The solution A and glacial acetic acid (150 mL) were added successively to the hydrogenation bottle and the cap was tightened. The hydrogenation bottle was replaced with nitrogen twice and then with hydrogen twice. The reaction was carried out by introducing hydrogen (15 psi of pressure) until the reaction was substantially completed. After completion of the reaction, the feed solution was subjected to diatomaceous earth to remove Pd/C. The filtrate was concentrated under reduced pressure to remove the solvent to give a residue. The residue was stirred with methyl tert-butyl ether (300 mL) for 2 hours and filtered by pump to obtain a filter cake. The filter cake was dried in vacuo to obtain 20.3g of the title compound (yield: 59%, purity: 95.4%). ¹H NMR (600MHz, Acetone-d₆) δ 7.75 (s, 1H), 7.53 (s, H), 3.96 (s, 3H). LCMS: 202.1 ([M+H]⁺).

In the present disclosure, relational terms such as "first" and "second" are used only to distinguish one entity or operation from another entity or operation, and do not necessarily require or imply any actual relationship or order.

It can be understood from above description that although specific embodiments of the present disclosure have been described for illustrative purposes, various modifications or improvements can be made by one skilled in the art without departing from the spirit and scope of the present disclosure. Such variations or modifications should fall within the scope of the claims appended to this disclosure.

## Claims

1. A process for preparing a compound of formula (VII), 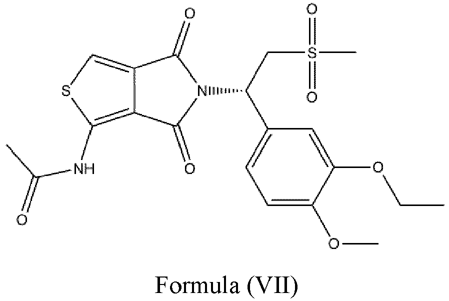 comprising subjecting a compound of formula (VI) or a compound of formula (VIII) to a condensation reaction

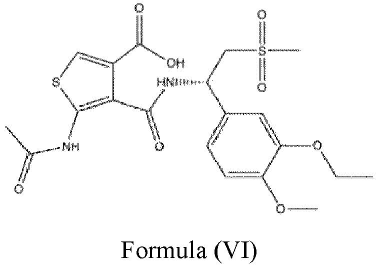

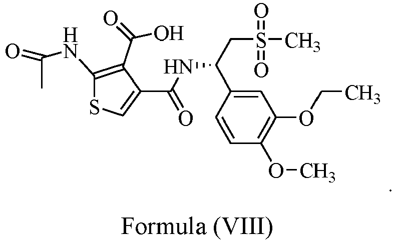

2. The process of claim 1, wherein the condensation reaction is carried out with a reagent selected from the group consisting of N,N'-carbonyldiimidazole (CDI), dicyclohexylcarbodiimide (DCC), 1-(3-dimethylaminopropyl) -3-ethylcarbodiimide (EDC), EDCI (EDC·HCl), dichlorosulfoxide (SOCl₂), oxalyl chloride ((COCl)₂), acetyl chloride (AcCl), acetic anhydride (Ac₂O), N,N'-dimethylformamide (DMF) and any mixture thereof, preferably with a reagent selected from the group consisting of N,N'-carbonyldiimidazole (CDI), dicyclohexylcarbodiimide (DCC), 1-(3-dimethylaminopropyl)-3-ethylcarbodiimide (EDC) or a salt thereof (1-(3-dimethylaminopropyl)-3-ethylcarbodiimide hydrochloride), oxalyl chloride ((COCl)₂), acetyl chloride (AcCl), acetic anhydride (Ac₂O), N,N-dimethylformamide (DMF) and any mixture thereof.

3. The process of claim 1 or 2, wherein a weight ratio of reagent to the compound of formula (VI) or the compound of formula (VIII) is from about 1:1 to about 3:1.

4. The process of any one of claims 1 to 3, wherein the condensation reaction is carried out in a solvent selected from the group consisting of tetrahydrofuran, dichloromethane (DCM), acetic anhydride (Ac₂O) and any mixture thereof.

5. The process of any one of claims 1 to 4, wherein the condensation reaction is carried out at about -5 to about 150°C.

6. The process of any one of claims 1 to 5, wherein the condensation reaction is carried out at about 20 to about 30°C.

7. The process of any one of claims 1 to 6, further comprising recrystallizing a resulting compound of formula (VII).

8. The method of claim 7, wherein a solvent for recrystallization is selected from the group consisting of acetonitrile/isopropanol, ethyl acetate, acetonitrile/water, tetrahydrofuran/water and any mixtures thereof.

9. A process for preparing a compound of formula (VI), 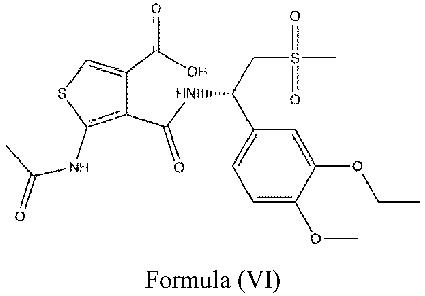 comprising subjecting a compound of formula (V) to a hydrolysis reaction

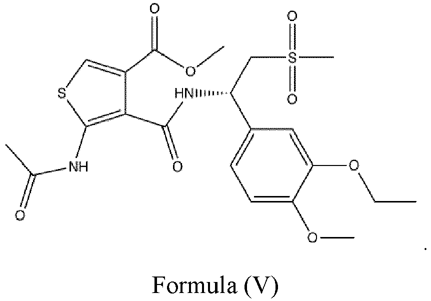

10. The process according to claim 9, wherein the hydrolysis reaction is carried out under alkaline conditions, preferably with potassium salt, sodium salt, lithium salt or any mixture thereof, more preferably with potassium hydroxide, potassium carbonate, potassium bicarbonate, sodium hydroxide, sodium carbonate, lithium hydroxide, lithium carbonate or any mixture thereof.

11. The process of claim 9 or 10, wherein the hydrolysis reaction is carried out at about -5 to about 35°C, preferably at about 0 to about 35°C, more preferably at about 20 to about 30°C.

12. The process of any one of claims 9 to 11, wherein the hydrolysis reaction is carried out in a solvent selected from the group consisting of ethanol/water, methanol/water, isopropanol/water, acetonitrile/acetone/water, acetonitrile/acetone, ethyl acetate, tetrahydrofuran, tetrahydrofuran/water, acetone/water and any mixture thereof.

13. The process of any one of claims 9 to 12, wherein a molar ratio of the compound of formula (V) to a reaction reagent of claim 10 is from about 1:1 to about 1:10, preferably from about 1:1 to about 1:6, more preferably from about 1:5.

14. A process for preparing a compound of formula (V), 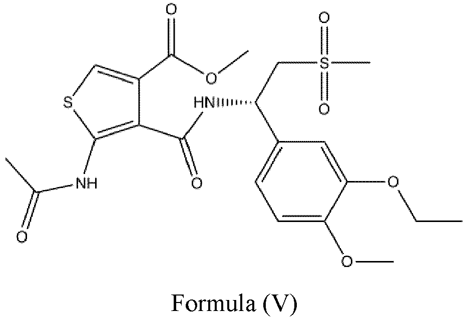 comprising subjecting a compound of formula (IV) to a condensation reaction

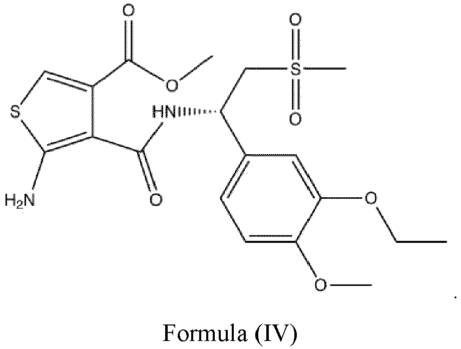

15. The process of claim 14, wherein the condensation reaction is carried out with a reagent selected from the group consisting of acetyl chloride (AcCl), acetic anhydride (Ac₂O) and any mixture thereof.

16. The process of claim 14 or 15, wherein the condensation reaction is carried out in a reagent selected from the group consisting of 4-dimethylaminopyridine (DMAP), N-methylmorpholine (NMM), triethylamine (TEA), N,N-diisopropylethylamine (DIEA), pyridine (Py) and any mixture thereof.

17. The process of any one of claims 14 to 16, wherein the condensation reaction is carried out in a reagent selected from the group consisting of Ac₂O, Ac₂O/DMAP, Ac₂O/DMAP/NMM, CH₃COCl/TEA/DCM/DMAP and CH₃COCl/Py.

18. The process of any one of claims 14 to 17, wherein the condensation reaction is carried out in a solvent selected from the group consisting of acetic anhydride, dichloromethane (DCM), pyridine (Py), tetrahydrofuran, acetonitrile, acetone, ethyl acetate and any mixture thereof.

19. The process of any one of claims 14 to 18, wherein the condensation reaction is carried out at about 0 to about 150°C, preferably at about 90 to about 150°C, more preferably at about 125 to about 140°C.

20. The process of any one of claims 14 to 19, wherein a solvent used to purify the compound of formula (V) is selected from the group consisting of ethanol, methyl tert-butyl ether (MTBE), methanol, ethyl acetate, n-hexane, diethyl ether, tetrahydrofuran (THF) and any mixture thereof.

21. A process for preparing a compound of formula (IV), 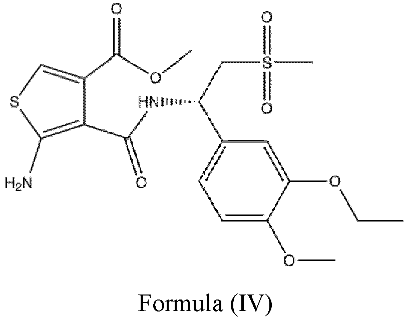 comprising subjecting a compound of formula (II) to a condensation reaction with a compound of formula (III) to obtain the compound of formula (IV)

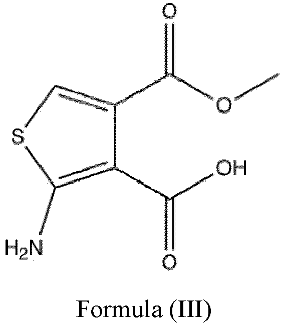

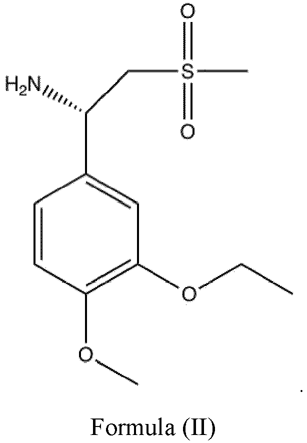

22. The process of claim 21, wherein the condensation reaction is carried out with a condensation agent selected from the group consisting of carbodiimide-type condensation agent, phosphorous cationic condensation agent, urea cationic condensation agent, other suitable condensation agents and any mixture thereof, wherein the carbodiimide-type condensation agent is preferably selected from the group consisting of 1-(3-dimethylaminopropyl)-3-ethylcarbodiimide (EDC), EDCI (EDC·HCl) and dicyclohexylcarbodiimide (DCC), the phosphorous cationic condensation agent is preferably selected from the group consisting of 1H-benzotriazole-1-yloxytrispyrrolidinophosphonium hexafluorophosphate (PyBOP), bromotrispyrrolidinophosphonium hexafluorophosphate (PyBrOP) and/or benzotriazol-1-yloxytris (dimethylamino) phosphonium hexafluorophosphate (BOP), the urea cationic condensation agent is preferably selected from the group consisting of N,N,N,N'-tetramethyl-O-(1H-benzotriazol-1-yl)uronium hexafluorophosphate (HBTU) and 2-(7-azabenzotriazol-1-yl))-N,N,N',N'-tetramethyluronium hexafluorophosphate (HATU), the other suitable condensing agents are preferably selected from the group consisting of 1-hydroxybenzotriazole (HOBt), 6-chloro-1-hydroxybenzotriazole (Cl-HOBt), N-hydroxy-7-azabenzotriazole (HOAt), bis(2-oxo-3-oxazolidinyl)phosphinic chloride (BOP-Cl), 3-diethoxyphosphoryloxy-1,2,3-benzotriazin-4(3H)-one (DEPBT), N-hydroxysuccinimide (HOSu) and/or pentafluorophenol.

23. The process of claim 21 or 22, wherein a solvent for carrying out the condensation reaction is selected from the group consisting of dichloromethane, trichloromethane, acetonitrile, toluene, tetrahydrofuran, dioxane and any mixture thereof.

24. The process of any one of claims 21 to 23, wherein the condensation reaction is carried out at about 0 to about 50°C, preferably at about 25 to about 35°C.

25. The process of any one of claims 21 to 24, wherein a weight ratio of an amount of the compound of formula (III) to an amount of the compound of formula (II) is from about 1:1.3 to about 1:1.4.

26. A process for preparing a compound of formula (III), 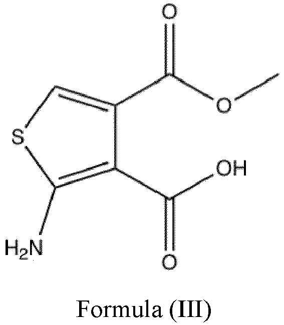 comprising reacting a compound of general formula (I) 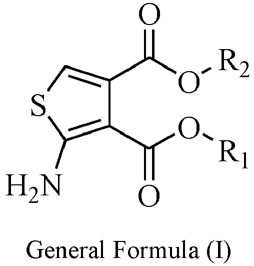 wherein R₁ ≠ R₂, R₁ and R₂ are selected from the group consisting of substituted or unsubstituted alkyl, substituted or unsubstituted alkenyl, and substituted or unsubstituted aryl, preferably R₁ ≠ R₂, R₁ and R₂ are each independently selected from the group consisting of substituted or unsubstituted C₁-C₆alkyl, substituted or unsubstituted C₂-C₆alkenyl, and substituted or unsubstituted C₆-C₁₂aryl, more preferably R₁ ≠ R₂, R₁ and R₂ are each independently selected from the group consisting of methyl, ethyl, propyl, tert-butyl, benzyl and allyl.

27. The process of claim 26, wherein the reaction is carried out in the presence of trifluoroacetic acid (TFA), hydrochloric acid, hydrobromic acid, sulfonic acid, palladium on carbon, tetrakistriphenylphosphine palladium or any mixture thereof.

28. The process according to claim 26 or 27, wherein the reaction is carried out at a weight ratio of trifluoroacetic acid (TFA), hydrochloric acid, hydrobromic acid, sulfonic acid, palladium on carbon, tetrakistriphenylphosphine palladium or any mixture thereof to the compound of formula (I) of about 1.5:1 to about 1:1, preferably at a ratio of trifluoroacetic acid (TFA), hydrochloric acid, hydrobromic acid, sulfonic acid, palladium on carbon, tetrakistriphenylphosphine palladium or any mixture thereof to the compound of formula (I) of about 1.1:1 to about 1:1.

29. The process of claim 26 or 28, wherein the solvent is selected from the group consisting of dichloromethane, toluene, dichloroethane, ethyl acetate, tetrahydrofuran, 1,4-dioxane, methanol, ethanol and any mixture thereof.

30. The process of any one of claims 26 to 29, wherein the hydrolysis reaction is carried out at about -20 to about 0°C, preferably at about -15 to about -5°C.

31. A process for preparing a compound of formula (VII) comprising
reacting a compound of general formula (I) to obtain a compound of formula (III);
subjecting the compound of formula (III) to a condensation reaction with a compound of formula (II) to obtain a compound of formula (IV);
subjecting the compound of formula (IV) to a condensation reaction to obtain a compound of formula (V);
subjecting the compound of formula (V) to a hydrolysis reaction to obtain a compound of formula (VI); and
subjecting the compound of formula (VI) or a compound of formula (VIII) to a condensation reaction to obtain the compound of Formula (VII).
